(19) Europäisches Patentamt
European Patent Office
Office européen des brevets

(11) **EP 1 972 630 A1**

(12) **EUROPEAN PATENT APPLICATION**

(43) Date of publication:
**24.09.2008 Bulletin 2008/39**

(21) Application number: **07381019.4**

(22) Date of filing: **02.03.2007**

(51) Int Cl.:
***C07D 473/34*** *(2006.01)* ***A61K 31/52*** *(2006.01)*
***A61P 33/06*** *(2006.01)*

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **GLAXO GROUP LIMITED**
**Greenford,**
**Middlesex UB6 0NN (GB)**

(72) Inventor: **The designation of the inventor has not yet been filed**

(74) Representative: **Elzaburu Marquez, Alberto**
**Elzaburu S.A.,**
**Miguel Angel, 21**
**28010 Madrid (ES)**

(54) **Purines as cysteine protease inhibitors**

(57) Substituted heteroaryl nitrile derivatives of Formula I,

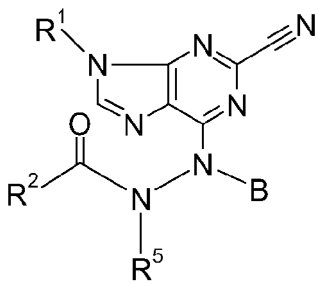

I

processes for their preparation, pharmaceutical compositions comprising such compounds and use of the compounds as cysteine protease inhibitors are provided.

EP 1 972 630 A1

## Description

## FIELD OF THE INVENTION

The invention is directed to certain substituted heteroaryl nitrile derivatives, which are protease inhibitors. More specifically, the compounds are inhibitors of cysteine proteases. In particular, the compounds inhibit cysteine proteases of the papain superfamily, more specifically those of the falcipain family, which are cysteine proteases found in the malaria parasite *Plasmodium falciparum.*

## BACKGROUND OF THE INVENTION

Malaria is one of the major disease problems of the developing world. The most virulent malaria-causing parasite in humans is *Plasmodium falciparum,* which is the cause of hundreds of millions of cases of malaria per annum, and is thought to cause over 1 million deaths each year, Breman, J. G., et al., (2001) Am. Trop. Med. Hyg. 64, 1-11. One problem encountered in the treatment of malaria is the build-up of resistance by the parasite to available drugs. Thus there is a need to develop new antimalarial drugs.

One way of identifying a potential new drug with antimalarial activity is to study biological targets found in the *Plasmodium falciparum* parasite, in turn by investigating biological pathways in which particular targets might be identified. In *Plasmodium falciparum,* haemoglobin is transported to an acidic food vacuole, where it is degraded. It appears that multiple enzymes, including food vacuole cysteine, aspartic, and metalloproteases, and a cytosolic aminopeptidase, contribute to haemoglobin hydrolysis, Francis S.E. et al., (1997) Annu. Rev. Microbiol. 51, 97-123; Rosenthal P.J. Protease inhibitors. In: Rosenthal P.J., ed. Antimalarial Chemotherapy: Mechanisms of Action, Resistance, and New Directions in Drug Discovery, Totowa, N.J.: Humana Press, (2001) 325-345. Plasmodial haemoglobinases are therefore potential therapeutic targets.

Cysteine protease inhibitors were shown some years ago to block haemoglobin degradation by erythrocytic parasites, causing a characteristic morphological abnormality in which the food vacuole fills with undegraded haemoglobin and parasite development is blocked, Rosenthal P. J., et al., (1998) J. Clin. Invest. 82, 1560-6; Gamboa de Dominguez N.D. and Rosenthal P.J., (1996) Blood 87, 4448-54. Efforts to identify enzymes responsible for haemoglobin degradation led to the characterization of "falcipain" as a trophozoite food vacuole cysteine protease, Rosenthal P.J. and Nelson R.G., (1992) Mol Biochem Parasitol 51, 143-52; Salas F. et al., (1995) Infect. Immun. 63 2120-5. It has more recently been found that "falcipain" actually constitutes three related papain-family cysteine proteases which share a number of unusual features, known as falcipain-1, falcipain-2 and falcipain-3, Rosenthal, P. J., et al., (2002) Curr. Pharm. Des. 8, 1659-1672. Falcipain-2 is the principal cysteine protease of *Plasmodium falciparum* trophozoites, Shenai B.R. et. al., (2000) J Biol Chem 275, 29000-10. Importantly, cysteine protease inhibitors that inhibit falcipain-2 consistently block haemoglobin hydrolysis and parasite development. These data suggest that falcipain-2 is a key target enzyme, but it is likely that the other two falcipains are also appropriate targets and that, in many cases, they are inhibited by the same compounds that are active against falcipain-2. Like falcipain-2, falcipain-3 readily hydrolyzes native haemoglobin under mildly reducing conditions that are similar to those found in physiological systems, Shenai B.R. et al., (2000) J. Biol. Chem. 275, 29000-10; Sijwali P.S. et al., (2001) Biochem. J. 360, 481-9; Shenai B.R. and Rosenthal P.J., (2002) Mol. Biochem. Parasitol. 122, 99-104. Falcipain-2 and falcipain-3 are similar in structure but falcipain-1 is a more distant relative; it is thought that this enzyme plays a key role in the invasion of erythrocytes by *Plasmodium falciparum* merozoites but that it is not essential for normal development during the erythrocytic stage, Sijwali, P. S., et al., Proceedings of the National Academy of Sciences of the United States of America 101, 8721-8726. Whether falcipain-1 also plays a role in haemoglobin processing is unknown. Very recently, a fourth papain-family cysteine protease has been found, now known as falcipain-2'. Falcipain-2' is nearly identical in sequence to falcipain-2, differing by only 3 amino acids, none of which are located at the active site. The structure of falcipain-2' is not known, but is likely to be very similar to that of falcipain-2. The biological role of falcipain-2' is also expected to be very similar, although probably not identical, to that of falcipain-2. In any event, cysteine protease inhibition, in particular the inhibition of falcipain-2, blocks parasite development. Falcipain-2 and related plasmodial cysteine proteases are thus logical targets for antimalarial chemotherapy and therefore there is a need for compounds which are inhibitors of these targets.

*P. vivax* is the second most important human malaria parasite, after *P. falciparum.* Although less virulent than *P. falciparum, P. vivax* is the most widely distributed human malaria parasite, and it causes extensive morbidity (Mendis, K., Sina, B. J., Marchesini, P. and Carter, R. (2001) "The neglected burden of Plasmodium vivax malaria" Am. J. Trop. Med. Hyg. 64, 97-106). These two parasites are responsible for more than 90% of episodes of human malaria, totalling several hundred million cases annually. However, comprehensive studies of *P. vivax* have been limited due to technical shortcomings. Notably, unlike the case with *P. falciparum,* routine *in vitro* culture of *P. vivax* is not available, and animal models are limited to primates. Very recently (Na, B.K., Shenai, B. R., Sijwali, P. S., Choe, Y., Pandey, K. C., Singh, A., Craik, C. S., Rosenthal, P. J. (2004) identification and biochemical characterization of vivapains, cysteine proteases of

the malaria parasite Plasmodium vivax. Biochem. J. 378, 529-538), two cysteine protease genes (vivapain-2 and vivapain-3) from *P. vivax* have been identified and cloned and the heterologously expressed gene products have been characterized biochemically. It was found that these cysteine proteases are apparent orthologues of falcipain-2 and falcipain-3, but key differences in the biochemical properties of the plasmodial proteases warrant attention to the inhibition of each enzyme in the evaluation of antimalarial protease inhibitors.

WO 2005/085210 A1 discloses certain fused bicyclic pyrimidine compounds as inhibitors of cathepsin K, useful in the treatment of bone diseases such as osteoporosis and the like. WO 2005/103012 A1 discloses certain hydrazine-heterocyclic nitrile compounds as inhibitors of cathepsin K, useful in the treatment of bone diseases such as osteoporosis and the like.

## SUMMARY OF THE INVENTION

The invention is directed to novel heteroaryl nitrile derivatives and their use as protease inhibitors, more specifically inhibitors of cysteine protease, even more specifically inhibitors of cysteine proteases of the papain superfamily. In one aspect of the invention the cysteine proteases are those of the falcipain family, for example falcipain-2 and falcipain-3, which are examples of cysteine proteases indicated in malaria.

The invention involves the compounds represented hereinbelow, pharmaceutical compositions comprising such compounds and use of the compounds as protease inhibitors.

## DETAILED DESCRIPTION OF THE INVENTION

The present invention provides a compound of Formula I:

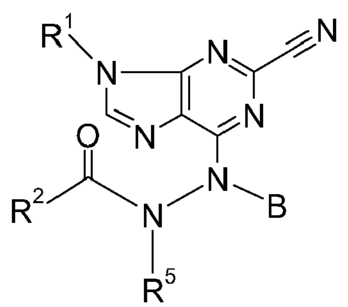

I

Wherein:

B represents

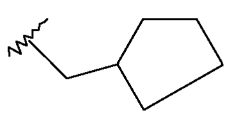

or

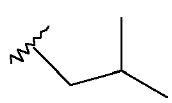

or

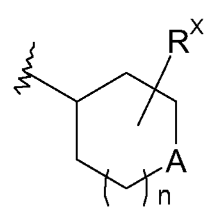

(i) (ii) (iii) ;

$R^1$ represents $C_{1-4}$alkyl, $C_{1-5}$alkylene-$NR^ER^F$ or $C_{1-2}$alkylene-N-phthalimide;

$R^5$ represents hydrogen or $C_{1-5}$alkylene-$NR^ER^F$;

$R^2$ represents -phenyl-$C_{1-3}$alkylene-X, -phenyl-$C_{1-3}$alkylene-X-$R^J$, -$O^tBu$, pyridyl-phenyl-$C_{1-3}$alkylene-X, or pyridyl-phenyl-$C_{1-3}$alkylene-X-$R^J$,
wherein phenyl is optionally substituted with one group selected from halogen or $CF_3$; $R^J$ represents Z, -$C_{1-3}$alkylene-Z or -C(O)Z;

X and Z independently represent a monocyclic 4-, 5- or 6-membered, saturated hydrocarbon group containing one or two nitrogen atoms, which is optionally substituted with a group selected from: $C_{1-4}$alkyl, $C_{1-4}$alkylOH, OH and $NR^ER^F$;

Either A represents $CH_2$ and n represents 0 or 1; or A represents -O- or N(C(O)$C_{1-3}$alkyl) and n represents 1;
When A represents $CH_2$ and n represents 0, $R^x$ represents an optional methyl substituent on any carbon atom of the ring to which it is attached, otherwise $R^x$ is absent;

$R^E$ and $R^F$ independently for each occurrence represent hydrogen or $C_{1-4}$alkyl;

Provided that at least one of $R^1$, $R^2$, $R^5$ or B contains at least one nitrogen atom;
or a pharmaceutically acceptable derivative thereof.
In one aspect of the invention, B represents

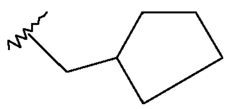

(i)

or

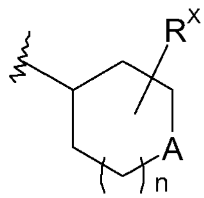

(iii) .

In another aspect, B represents

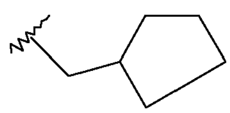

(i)

In a further aspect, B represents

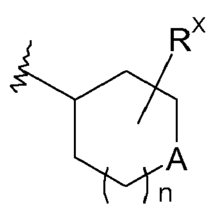

(iii)

In a yet further aspect, B represents

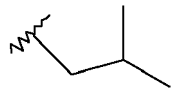

(ii)

In one aspect of the invention, $R^1$ represents $C_{1-4}$alkyl or $C_{1-5}$alkylene-$NR^ER^F$.
In one aspect of the invention, $R^5$ represents hydrogen.
In one aspect of the invention, $R^2$ represents -phenyl-$C_{1-3}$alkylene-X, -phenyl-$C_{1-3}$alkylene-X-$R^J$ or -$O^tBu$. In another aspect, $R^2$ represents -phenyl-$C_{1-3}$alkylene-X-$R^J$ or -$O^tBu$. In a further aspect, $R^2$ represents -phenyl-$C_{1-3}$alkylene-X-$R^J$.
In one aspect of the invention, X represents an optionally substituted monocyclic 4- or 6-membered, saturated hydrocarbon group containing one or two nitrogen atoms. In another aspect, X represents an optionally substituted monocyclic 6-membered, saturated hydrocarbon group containing one or two nitrogen atoms. In a further aspect, X represents piperidine or piperazine. In a yet further aspect, X is unsubstituted.
In another aspect, Z represents an optionally substituted monocyclic 5- or 6-membered, saturated hydrocarbon group containing one or two nitrogen atoms. In another aspect, Z represents an optionally substituted monocyclic 6-membered, saturated hydrocarbon group containing one or two nitrogen atoms. In a further aspect, Z represents piperidine or piperazine. In a yet further aspect, Z is unsubstituted.
In one aspect of the invention, A represents $CH_2$ and n represents 0 or 1. In another aspect of the invention when A represents $CH_2$, n represents 0. In a further aspect, when A represents $CH_2$, n represents 1. In a yet further aspect, A represents -O- or N(C(O)$C_{1-3}$alkyl).
In one aspect of the invention when A represents $CH_2$ and n represents 0, $R^X$ is absent.
In one aspect of the invention, $R^E$ and $R^F$ independently for each occurrence represent $C_{1-4}$alkyl.
The meaning of any functional group or substituent thereon at any one occurrence in Formula I or any subformula thereof, is independent of its meaning, or any other functional group's or substituent's meaning, at any other occurrence, unless stated otherwise.
It is to be understood that the present invention covers all combinations of the groups according to different aspects of the invention as described hereinabove.

**Terms and Definitions**

As used herein, the term "alkyl" as a group or a part of a group refers to a linear or branched alkyl group containing the indicated number of carbon atoms. Examples of such groups include methyl, ethyl, n-propyl, isopropyl, n-butyl, isobutyl, sec-butyl, tert-butyl, and the like.
As used herein, the term "alkylene" as a group or a part of a group refers to a linear or branched saturated hydrocarbon linker group containing the indicated number of carbon atoms. Examples of such groups include methylene, ethylene and the like.
As used herein, the term "halogen" or "halo" refers to a fluorine (fluoro), chlorine (chloro), bromine (bromo) or iodine (iodo) atom.
As used herein, the term "proteases" are enzymes that catalyze the cleavage of amide bonds of peptides and proteins by nucleophilic substitution at the amide bond, ultimately resulting in hydrolysis. Proteases include: cysteine proteases, serine proteases, aspartic proteases, and metalloproteases. Protease "inhibitors" bind more strongly to the enzyme than the substrate and in general are not subject to cleavage after enzyme catalyzed attack by the nucleophile. They therefore competitively prevent proteases from recognizing and hydrolysing natural substrates and thereby act as inhibitors.
In one aspect of the invention there is provided a compound selected from the list:

*N'*-(2-cyano-9-methyl-9*H*-purin-6-yl)-*N'*-(cyclopentylmethyl)-4-{[4-(4-methyl-1-piperazinyl)-1-piperidinyl]methyl} benzohydrazide;
*N'*-(2-cyano-9-methyl-9*H*-purin-6-yl)-*N'*-(cyclopentylmethyl)-4-({4-[(1-methyl-4-piperidinyl)methyl]-1-piperazinyl} methyl)benzohydrazide;
*N'*-(2-cyano-9-methyl-9*H*-purin-6-yl)-*N'*-(cyclopentylmethyl)-4-({4-[(1-methyl-3-piperidinyl)methyl]-1-piperazinyl} methyl)benzohydrazide;

*N*'-(2-cyano-9-methyl-9*H*-purin-6-yl)-*N*'-cyclopentyl-4-{[4-(4-methyl-1-piperazinyl)-1-piperidinyl]methyl}benzohydrazide;
*N*'-(2-cyano-9-methyl-9*H*-purin-6-yl)-*N*'-cyclopentyl-4-({4-[(1-methyl-4-piperidinyl)methyl]-1-piperazinyl}methyl) benzohydrazide;
*N*'-(2-cyano-9-methyl-9*H*-purin-6-yl)-*N*'-cyclopentyl-4-({4-[(4-methyl-1-piperazinyl)carbonyl]-1-piperidinyl}methyl) benzohydrazide;
*N*'-(2-cyano-9-methyl-9*H*-purin-6-yl)-*N*'-cyclopentyl-4-{[3-(1-pyrrolidinyl)-1-azetidinyl]methyl}benzohydrazide;
*N*'-(2-cyano-9-methyl-9*H*-purin-6-yl)-*N*'-cyclohexyl-4-{[4-(4-methyl-1-piperazinyl)-1-piperidinyl]methyl}benzohydrazide;
*N*'-(2-cyano-9-methyl-9*H*-purin-6-yl)-*N*'-cyclohexyl-4-({4-[(1-methyl-4-piperidinyl)methyl]-1-piperazinyl}methyl)-benzohydrazide;
*N*'-(2-cyano-9-methyl-9*H*-purin-6-yl)-*N*'-cyclohexyl-4-({4-[(4-methyl-1-piperazinyl)carbonyl]-1-piperidinyl}methyl) benzohydrazide;
*N'*-(2-cyano-9-methyl-9*H*-purin-6-yl)-*N'*-cyclohexyl-4-{[3-(1-pyrrolidinyl)-1-azetidinyl]methyl}benzohydrazide;
1,1-dimethylethyl 2-{2-cyano-9-[3-(dimethylamino)propyl]-9*H*-purin-6-yl}-2-(cyclopentylmethyl)hydrazinecarboxylate;
1,1-dimethylethyl 2-{2-cyano-9-[2-(dimethylamino)ethyl]-9*H*-purin-6-yl}-2-(cyclopentylmethyl)hydrazinecarboxylate;
*N'*-{2-cyano-9-[2-(dimethylamino)ethyl]-9*H*-purin-6-yl}-*N'*-(cyclo pentylmethyl)-4-[(4-methyl-1-piperazinyl)methyl] benzohydrazide;
1,1-dimethylethyl 2-{2-cyano-9-[3-(dimethylamino)propyl]-9*H*-purin-6-yl}-2-cyclopentylhydrazinecarboxylate;
1,1-dimethylethyl 2-{2-cyano-9-[3-(dimethylamino)propyl]-9*H*-purin-6-yl}-2-(cyclopentylmethyl)-1-[3-(dimethylamino)propyl]hydrazinecarboxylate;
*N*'-{2-cyano-9-[3-(dimethylamino)propyl]-9*H*-purin-6-yl}-*N*'-cyclopentyl-4-[(4-methyl-1-piperazinyl)methyl]benzohydrazide;

and pharmaceutically acceptable derivatives thereof.

As used herein, the term "pharmaceutically acceptable derivative", means any pharmaceutically acceptable salt, solvate, or prodrug e.g. ester or carbamate of a compound of Formula I, which upon administration to the recipient is capable of providing (directly or indirectly) a compound of Formula I, or an active metabolite or residue thereof. Such derivatives are recognizable to those skilled in the art, without undue experimentation. Nevertheless, reference is made to the teaching of Burger's Medicinal Chemistry and Drug Discovery, 5th Edition, Vol 1: Principles and Practice, which is incorporated herein by reference to the extent of teaching such derivatives. In one aspect of the invention pharmaceutically acceptable derivatives are salts, solvates, esters and carbamates. In another aspect of the invention pharmaceutically acceptable derivatives are salts, solvates and esters. In a further aspect, pharmaceutically acceptable derivatives are salts and solvates.

The compounds of the present invention may be in the form of and/or may be administered as a pharmaceutically acceptable salt. Indeed, in certain embodiments of the invention, pharmaceutically acceptable salts of the compounds according to Formula I, may be preferred over the respective free base or free acid because such salts impart greater stability or solubility to the molecule thereby facilitating formulation into a dosage form. Accordingly, the invention is further directed to pharmaceutically acceptable salts of the compounds according to Formula I.

As used herein, the term "pharmaceutically acceptable salts" refers to salts that retain the desired biological activity of the subject compound and exhibit minimal undesired toxicological effects. For a review on suitable salts see Berge et al, J. Pharm. Sci., 1977, 66, 1-19. The term "pharmaceutically acceptable salts" includes both pharmaceutically acceptable acid addition salts and pharmaceutically acceptable base addition salts. These pharmaceutically acceptable salts may be prepared *in situ* during the final isolation and purification of the compound, or by separately reacting the purified compound in its free acid or free base form with a suitable base or acid, respectively. The salt may precipitate from solution and be collected by filtration or may be recovered by evaporation of the solvent.

A pharmaceutically acceptable acid addition salt can be formed by reaction of a compound of Formula I, with a suitable inorganic or organic acid (such as hydrobromic, hydrochloric, sulfuric, sulfamic, nitric, phosphoric, succinic, maleic, hydroxymaleic, acrylic, formic, acetic, hydroxyacetic, phenylacetic, butyric, isobutyric, propionic, fumaric, citric, tartaric, lactic, mandelic, benzoic, o-acetoxybenzoic, chlorobenzoic, methylbenzoic, dinitrobenzoic, hydroxybenzoic, methoxybenzoic salicylic, glutamaic, stearic, ascorbic, palmitic, oleic, pyruvic, pamoic, malonic, lauric, glutaric aspartic, p-toluenesulfonic, benzenesulfonic, methanesulfonic, ethanesulfonic, 2-hydroxyethanesulfonic, naphthalenesulfonic (e.g. 2-naphthalenesulfonic), p-aminobenzenesulfonic (i.e. sulfanilic), hexanoic, heptanoic, or phthalic acid), optionally in a suitable solvent such as an organic solvent, to give the salt which is usually isolated for example by crystallisation and filtration. A pharmaceutically acceptable acid addition salt of a compound of Formula I, can comprise or be for example a hydrobromide, hydrochloride, hydroiodide, sulfate, bisulfate, nitrate, phosphate, hydrogen phosphate, succinate, maleate, malate, formate, acetate, trifluoroacetate, saccharate, propionate, fumarate, citrate, tartrate, lactate, benzoate, salicylate, glutamate, aspartate, p-toluenesulfonate, benzenesulfonate, methanesulfonate, ethanesulfonate, naphthalenesulfonate (e.g. 2- naphthalenesulfonate), methanesulphonic, ethanesulphonic, p-toluenesulphonic, isethionate or hexanoate salt. In one embodiment there is provided the trifluoroacetic acid salts of the compounds of the invention. In another embodiment there is provided the hydrochloric acid salts of the compounds of the invention.

A pharmaceutically acceptable base addition salt can be formed by reaction of a compound of Formula I with a suitable inorganic or organic base (e.g. ammonia, triethylamine, ethanolamine, triethanolamine, choline, arginine, lysine or histidine), optionally in a suitable solvent such as an organic solvent, to give the base addition salt which is usually isolated for example by crystallisation and filtration. Pharmaceutically acceptable base salts include ammonium salts and salts with organic bases, including salts of primary, secondary and tertiary amines, including aliphatic amines, aromatic amines, aliphatic diamines, and hydroxy alkylamines, such as methylamine, ethylamine, isopropylamine, diethylamine, ethylenediamine, ethanolamine, trimethylamine, dicyclohexyl amine, diethanolamine, cyclohexylamine and N-methyl-D-glucamine. Other suitable pharmaceutically acceptable base salts include pharmaceutically acceptable metal salts, for example pharmaceutically acceptable alkali-metal or alkaline-earth-metal salts such as hydroxides, carbonates and bicarbonates of sodium, potassium, lithium, calcium, magnesium, aluminium, and zinc; in particular pharmaceutically acceptable metal salts of one or more carboxylic acid moieties that may be present in the compound of Formula I.

Other non-pharmaceutically acceptable salts, for example oxalates may be used, for example in the isolation of compounds of the invention.

The invention includes within its scope all possible stoichiometric and non-stoichiometric forms of the salts of the compounds of Formula I.

As used herein, the term "compounds of the invention" means the compounds according to Formula I, and the pharmaceutically acceptable derivatives thereof. The term "a compound of the invention" means any one of the compounds of the invention as defined above.

The compounds of the invention may exist as solids or liquids, both of which are included in the invention. In the solid state, the compounds of the invention may exist as either amorphous material or in crystalline form, or as a mixture thereof. It will be appreciated that solvates of the compounds of the invention may be formed wherein solvent molecules are incorporated into the crystalline lattice during crystallisation. Solvates may involve non-aqueous solvents such as ethanol, isopropanol, DMSO, acetic acid, ethanolamine, and ethyl acetate, or they may involve water as the solvent that is incorporated into the crystalline lattice. Solvates wherein water is the solvent that is incorporated into the crystalline lattice are typically referred to as "hydrates." The invention includes all such solvates.

It will be further appreciated that all crystalline forms, polymorphs, geometric isomers, stereoisomers (including enantiomers and diastereomers) and tautomers of the compounds of the invention, or mixtures thereof, are contemplated to be within the scope of the present invention. Unless otherwise specfied, for compounds which posesses stereocentres and which can therefore form enantiomers, the compound contains a 1:1 mixture of enantiomers, i.e. a racemic mixture of enantiomers.

According to another aspect of the invention there is provided a compound of Formula I, or a pharmaceutically acceptable derivative thereof for use in human or veterinary medical therapy.

The compounds of the invention may be useful for treating conditions in which cysteine proteases are implicated, including infections by *Plasmodium falciparum* which is the most virulent malaria-causing parasite, and by *Plasmodium vivax, Pneumocystis carinii, Trypsanoma cruzi, Trypsanoma brucei,* and *Crithidia fusiculata;* as well as in treating conditions such as malaria. Accordingly, the invention is directed to methods of treating such conditions.

In one aspect of the invention, there is provided a compound of Formula I, or a pharmaceutically acceptable derivative thereof for use in the treatment of a condition mediated by inhibition of a cysteine protease, particularly inhibition of a cysteine protease of the papain superfamily such as those of the falcipain family, including falcipain-2 or falcipain-3, for example malaria.

In another aspect of the invention there is provided the use of a compound of Formula I, or a pharmaceutically acceptable derivative thereof in the manufacture of a medicament for the treatment of a condition mediated by inhibition of a cysteine protease, particularly inhibition of a cysteine protease of the papain superfamily such as those of the falcipain family, including falcipain-2 or falcipain-3, for example malaria.

In another aspect of the invention there is provided a method for the treatment of a human or animal subject suffering from a condition mediated by inhibition of a cysteine protease, particularly inhibition of a cysteine protease of the papain superfamily such as those of the falcipain family, including falcipain-2 or falcipain-3, for example malaria, which method comprises administering an effective amount of a compound of Formula I, or a pharmaceutically acceptable derivative thereof or a pharmaceutical composition comprising a compound of Formula I, or a pharmaceutically acceptable derivative thereof.

The compounds of the invention are cysteine protease inhibitors and can be useful in the treatment of a condition mediated by inhibition of a cysteine protease, particularly inhibition of a cysteine protease of the papain superfamily

such as those of the falcipain family, including falcipain-2 or falcipain-3, for example in the treatment of malaria.. Accordingly, the invention is further directed to pharmaceutical compositions comprising a compound of Formula I, or a pharmaceutically acceptable derivative thereof.

The methods of treatment of the invention comprise administering a safe and effective amount of a compound of Formula I, or a pharmaceutically acceptable derivative thereof, or a pharmaceutical composition containing a compound of Formula I, or a pharmaceutically acceptable derivative thereof, to a patient in need thereof.

As used herein, "treatment" means: (1) the amelioration or prevention of the condition being treated or one or more of the biological manifestations of the condition being treated, (2) the interference with (a) one or more points in the biological cascade that leads to or is responsible for the condition being treated or (b) one or more of the biological manifestations of the condition being treated, or (3) the alleviation of one or more of the symptoms or effects associated with the condition being treated. The skilled artisan will appreciate that "prevention" is not an absolute term. In medicine, "prevention" is understood to refer to the prophylactic administration of a drug to substantially diminish the likelihood or severity of a condition or biological manifestation thereof, or to delay the onset of such condition or biological manifestation thereof.

As used herein, "safe and effective amount" means an amount of the compound sufficient to significantly induce a positive modification in the condition to be treated but low enough to avoid serious side effects (at a reasonable benefit/risk ratio) within the scope of sound medical judgment. A safe and effective amount of a compound of the invention will vary with the particular compound chosen (e.g. depending on the potency, efficacy, and half-life of the compound); the route of administration chosen; the condition being treated; the severity of the condition being treated; the age, size, weight, and physical condition of the patient being treated; the medical history of the patient to be treated; the duration of the treatment; the nature of concurrent therapy; the desired therapeutic effect; and like factors, but can nevertheless be routinely determined by the skilled artisan.

As used herein, "patient" refers to a human or other animal.

The compounds of the invention may be administered by any suitable route of administration, including both systemic administration and topical administration. Systemic administration includes oral administration, parenteral administration, transdermal administration, rectal administration, and administration by inhalation. Parenteral administration refers to routes of administration other than enteral, transdermal, or by inhalation, and is typically by injection or infusion. Parenteral administration includes intravenous, intramuscular, and subcutaneous injection or infusion. Inhalation refers to administration into the patient’s lungs whether inhaled through the mouth or through the nasal passages. Topical administration includes application to the skin as well as intraocular, optic, intravaginal, and intranasal administration.

The compounds of the invention may be administered once or according to a dosing regimen wherein a number of doses are administered at varying intervals of time for a given period of time. For example, doses may be administered one, two, three, or four times per day. Doses may be administered until the desired therapeutic effect is achieved or indefinitely to maintain the desired therapeutic effect. Suitable dosing regimens for a compound of the invention depend on the pharmacokinetic properties of that compound, such as absorption, distribution, and half-life, which can be determined by the skilled artisan. In addition, suitable dosing regimens, including the duration such regimens are administered, for a compound of the invention depend on the condition being treated, the severity of the condition being treated, the age and physical condition of the patient being treated, the medical history of the patient to be treated, the nature of concurrent therapy, the desired therapeutic effect, and like factors within the knowledge and expertise of the skilled artisan. It will be further understood by such skilled artisans that suitable dosing regimens may require adjustment given an individual patient’s response to the dosing regimen or over time as individual patient needs change.

Typical daily dosages may vary depending upon the particular route of administration chosen. Typical daily dosages for oral administration range from about 0.01 to about 25 mg/kg, in one embodiment from about 0.1 to about 14 mg/kg. Typical daily dosages for parenteral administration range from about 0.001 to about 10 mg/kg; in one embodiment from about 0.01 to about 6 mg/kg .The compounds of Formula I, may also be used in combination with other therapeutic agents. The invention thus provides, in a further aspect, a combination comprising a compound of Formula I, or a pharmaceutically acceptable derivative thereof together with a further therapeutic agent. When a compound of Formula I, or a pharmaceutically acceptable derivative thereof is used in combination with a second therapeutic agent active against the same disease state the dose of each compound may differ from that when the compound is used alone. Appropriate doses will be readily appreciated by those skilled in the art. It will be appreciated that the amount of a compound of the invention required for use in treatment will vary with the nature of the condition being treated and the age and the condition of the patient and will be ultimately at the discretion of the attendant physician or veterinarian.

The compounds of the present invention may be used alone or in combination with one or more additional active agents, such as other inhibitors of cysteine proteases or antimalarial drugs.

Such additional active agents include antimalarial drugs, such as folates (e.g. chloroquine, mefloquine, primaquine pyrimethamine, quinine artemisinin, halofantrine, doxycycline, amodiquine, atovaquine [atovaquone], tafenoquine) and antifolates (e.g. dapsone, proguanil, sulfadoxine, pyrimethamine, chlorcycloguanil, cycloguanil) or antibacterial drugs such as azithromycin, doxycycline, ciprofloxacin and clindamycin.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations by any convenient route.

When administration is sequential, either the compound of the present invention or the second therapeutic agent may be administered first. When administration is simultaneous, the combination may be administered either in the same or different pharmaceutical composition. When combined in the same formulation it will be appreciated that the two compounds must be stable and compatible with each other and the other components of the formulation. When formulated separately they may be provided in any convenient formulation, conveniently in such manner as are known for such compounds in the art.

**Compositions**

The compounds of the invention will normally, but not necessarily, be formulated into pharmaceutical compositions prior to administration to a patient. In one aspect, the invention is directed to pharmaceutical compositions comprising a compound of the invention. In another aspect the invention is directed to pharmaceutical compositions comprising a compound of the invention and a pharmaceutically acceptable carrier and/or excipient. The carrier and/or excipient must be "acceptable" in the sense of being compatible with the other ingredients of the formulation and not deleterious to the receipient thereof.

The pharmaceutical compositions of the invention may be prepared and packaged in bulk form wherein a safe and effective amount of a compound of the invention can be extracted and then given to the patient such as with powders or syrups. Alternatively, the pharmaceutical compositions of the invention may be prepared and packaged in unit dosage form wherein each physically discrete unit contains a safe and effective amount of a compound of the invention. When prepared in unit dosage form, the pharmaceutical compositions of the invention typically contain from about 0.5 mg to about 1750 mg, e.g. from about 5 mg to about 1000 mg for oral dosage forms and from about 0.05 mg to about 700 mg, e.g. from about 0.5 mg to about 500 mg for parenteral dosage forms.

The pharmaceutical compositions of the invention typically contain one compound of the invention. However, in certain embodiments, the pharmaceutical compositions of the invention contain more than one compound of the invention. For example, in certain embodiments the pharmaceutical compositions of the invention contain two compounds of the invention. In addition, the pharmaceutical compositions of the invention may optionally further comprise one or more additional pharmaceutically active compounds. Conversely, the pharmaceutical compositions of the invention typically contain more than one pharmaceutically acceptable excipient. However, in certain embodiments, the pharmaceutical compositions of the invention contain one pharmaceutically acceptable excipient.

As used herein, the term "pharmaceutically acceptable" means suitable for pharmaceutical use.

The compound of the invention and the pharmaceutically acceptable excipient or excipients will typically be formulated into a dosage form adapted for administration to the patient by the desired route of administration. For example, dosage forms include those adapted for (1) oral administration such as tablets, capsules, caplets, pills, troches, powders, syrups, elixers, suspensions, solutions, emulsions, sachets, and cachets; (2) parenteral administration such as sterile solutions, suspensions, and powders for reconstitution; (3) transdermal administration such as transdermal patches; (4) rectal administration such as suppositories; (5) inhalation such as aerosols and solutions; and (6) topical administration such as creams, ointments, lotions, solutions, pastes, sprays, foams, and gels.

Suitable pharmaceutically acceptable excipients will vary depending upon the particular dosage form chosen. In addition, suitable pharmaceutically acceptable excipients may be chosen for a particular function that they may serve in the composition. For example, certain pharmaceutically acceptable excipients may be chosen for their ability to facilitate the production of uniform dosage forms. Certain pharmaceutically acceptable excipients may be chosen for their ability to facilitate the production of stable dosage forms. Certain pharmaceutically acceptable excipients may be chosen for their ability to facilitate the carrying or transporting the compound or compounds of the invention once administered to the patient from one organ, or portion of the body, to another organ, or portion of the body. Certain pharmaceutically acceptable excipients may be chosen for their ability to enhance patient compliance.

Suitable pharmaceutically acceptable excipients include the following types of excipients: binders, disintegrants, lubricants, glidants, granulating agents, coating agents, wetting agents, solvents, co-solvents, suspending agents, emulsifiers, sweeteners, flavoring agents, flavor masking agents, coloring agents, anticaking agents, humectants, chelating agents, plasticizers, viscosity increasing agents, antioxidants, preservatives, stabilizers, surfactants, and buffering agents. The skilled artisan will appreciate that certain pharmaceutically acceptable excipients may serve more than one function and may serve alternative functions depending on how much of the excipient is present in the formulation and what other ingredients are present in the formulation.

Skilled artisans possess the knowledge and skill in the art to enable them to select suitable pharmaceutically

acceptable excipients in appropriate amounts for use in the invention. In addition, there are a number of resources that are available to the skilled artisan which describe pharmaceutically acceptable excipients and may be useful in selecting suitable pharmaceutically acceptable excipients. Examples include Remington's Pharmaceutical Sciences (Mack Publishing Company), The Handbook of Pharmaceutical Additives (Gower Publishing Limited), and The Handbook of Pharmaceutical Excipients (the American Pharmaceutical Association and the Pharmaceutical Press).

The pharmaceutical compositions of the invention are prepared using techniques and methods known to those skilled in the art. Some of the methods commonly used in the art are described in Remington's Pharmaceutical Sciences (Mack Publishing Company).

In one aspect, the invention is directed to a solid or liquid oral dosage form such as a liquid, tablet, lozenge or a capsule, comprising a safe and effective amount of a compound of the invention and a carrier. The carrier may be in the form of a diluent or filler. Suitable diluents and fillers in general include lactose, sucrose, dextrose, mannitol, sorbitol, starch (e.g. corn starch, potato starch, and pre-gelatinized starch), cellulose and its derivatives (e.g. microcrystalline cellulose), calcium sulfate, and dibasic calcium phosphate. A liquid dosage form will generally consist of a suspension or solution of the compound or salt in a liquid carrier for example, ethanol, olive oil, glycerine, glucose (syrup) or water (e.g. with an added flavouring, suspending, or colouring agent). Where the composition is in the form of a tablet or lozenge, any pharmaceutical carrier routinely used for preparing solid formulations may be used. Examples of such carriers include magnesium stearate, terra alba, talc, gelatin, acacia, stearic acid, starch, lactose and sucrose. Where the composition is in the form of a capsule, any routine encapsulation is suitable, for example using the aforementioned carriers or a semi solid e.g. mono di-glycerides of capric acid, Gelucire™ and Labrasol™, or a hard capsule shell e.g gelatin. Where the composition is in the form of a soft shell capsule e.g. gelatin, any pharmaceutical carrier routinely used for preparing dispersions or suspensions may be considered, for example aqueous gums or oils, and may be incorporated in a soft capsule shell.

An oral solid dosage form may further comprise an excipient in the form of a binder. Suitable binders include starch (e.g. corn starch, potato starch, and pre-gelatinized starch), gelatin, acacia, sodium alginate, alginic acid, tragacanth, guar gum, povidone, and cellulose and its derivatives (e.g. microcrystalline cellulose). The oral solid dosage form may further comprise an excipient in the form of a disintegrant. Suitable disintegrants include crospovidone, sodium starch glycolate, croscarmelose, alginic acid, and sodium carboxymethyl cellulose. The oral solid dosage form may further comprise an excipient in the form of a lubricant. Suitable lubricants include stearic acid, magnesium stearate, calcium stearate, and talc.

There is further provided by the present invention a process of preparing a pharmaceutical composition, which process comprises mixing a compound of Formula I, or a pharmaceutically acceptable derivative thereof, together with a pharmaceutically acceptable carrier and/or excipient.

Preparations for oral administration may be suitably formulated to give controlled/extended release of the active compound.

All publications, including but not limited to patents and patent applications cited in this specification are herein incorporated by reference as if each individual publication were specifically and individually indicated to be incorporated by reference as though fully set forth.

**Abbreviations**

In describing the invention, chemical elements are identified in accordance with the Periodic Table of the Elements. Abbreviations and symbols utilized herein are in accordance with the common usage of such abbreviations and symbols by those skilled in the chemical arts. The following abbreviations are used herein:

| | |
|---|---|
| ACN | acetonitrile |
| AcOEt / AcOEt | ethyl acetate |
| AcOH | acetic acid |
| AFC | 7-amido-4-trifluoromethylcoumarin |
| AMC | 7-amido-4-methylcoumarin |
| anh. | anhydrous |
| aq. | aqueous |
| ˚C | degrees Celcius/centigrade |
| cat. | catalytic |
| $CDCl_3$ | deuterated chloroform |
| CHAPS | 3-[(3-cholamidopropyl)dimethylammonio]-1-propanesulfonate |
| CYS | cysteine |
| DABCO | 1,4-diazabicyclo[2.2.2]octane |
| dba | dibenzylideneacetone |

| | |
|---|---|
| DCM | dichloromethane |
| DHP | 3,4-dihydro-2H-pyran |
| DIPEA | diisopropylamine |
| DMA / DMAC | dimethyacetamide |
| DMF | dimethylformamide |
| DMSO-d6 | deuterated dimethylsulfoxide |
| DMSO | dimethylsulfoxide |
| dppf | 1,1'-bis-(diphenylphosphino)ferrocene |
| DTT | dithiothreitol |
| E64 | *trans*-epoxysuccinyl-L-leucylamido(4-guanidino)butane |
| EDCI | N-(3-dimethylaminopropyl)-N'-ethylcarbodiimide |
| EDTA | (ethylenedinitrilo)tetraacetic acid |
| ES+ MS | Positive Electrospray mass spectrometry |
| ES- MS | Negative Electrospray mass spectrometry |
| EtOH | ethanol |
| g | gramme(s) |
| h | hour(s) |
| H-D-VLR-AFC | HD-Valyl-Leucyl-Arginyl-7-Amido-4-trifluoromethylcoumarin |
| Hex | hexane (mixture of hexanes) |
| $^1$H-NMR | proton nuclear magnetic resonance |
| HPLC | high performance liquid chromatography |
| i-PrOH | isopropanol |
| kg | kilogram(s) |
| KQKLR-AMC | N-acetyl-Lysyl-Glutaminyl-Lysyl-Leucyl-Arginyl-7-Amido-4-methylcoumarin |
| MeOH | methanol |
| MES | 2-(N-morpholino)ethanesulfonic acid |
| MHz | megahertz |
| min | minute(s) |
| mg | miligram(s) |
| mL | mililitre(s) |
| mm | milimetre(s) |
| mmol | milimole(s) |
| μL | microlitre(s) |
| nM | nanomolar |
| NMR | nuclear magnetic resonance |
| $Pd_2(dba)_3$/Zn | tris(dibenzylideneacetone)dipalladium(0)/zinc |
| ppm | parts per million |
| r.t. | room temperature |
| s | second(s) |
| sat. | saturated |
| tBu | *tert*-butyl |
| TEA | triethylamine |
| TFA | trifluoroacetic acid |
| THF | tetrahydrofuran |
| TsOH | 4-toluenesulfonic acid |
| Z-LR-AMC | benzyloxycarbonyl-leucyl-arginyl-7-amido-4-methylcoumarin |

**Compound Preparation**

The general procedures used to synthesise the compounds of Formula I are described in reaction Schemes 1-13 and are illustrated in the Examples.

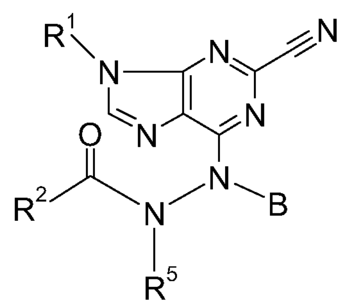

I

Compounds of Formula I wherein $R^5$ is hydrogen may be prepared from a reaction between compounds of Formula II, wherein $R^1$ and B are as defined for Formula I, compounds of Formula III, wherein Hal is chlorine, bromine or iodine, and compounds of Formula IV, wherein X and $R^J$ are as defined for Formula I, according to Scheme 1. Compounds II are reacted with compounds III in the presence of a suitable base, such as DIPEA or $K_2CO_3$, in a suitable solvent such as THF or ACN, followed by the addition of compounds IV, to give compounds I.

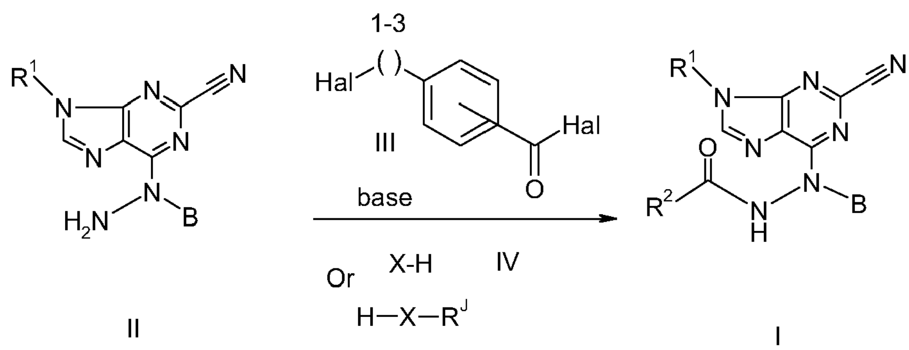


II

I

Scheme 1

Alternatively, compounds of Formula I, wherein $R^5$ is hydrogen, may be prepared from a reaction between compounds of Formula V, wherein $R^1$ and B are as defined for Formula I and Hal is chlorine, bromine or iodine, and compounds of Formula IV, wherein X and $R^J$ are as defined for Formula I, according to Scheme 2. Compounds V are reacted with IV in the presence of a suitable base such as DIPEA or $K_2CO_3$, in a suitable solvent such as THF or ACN.

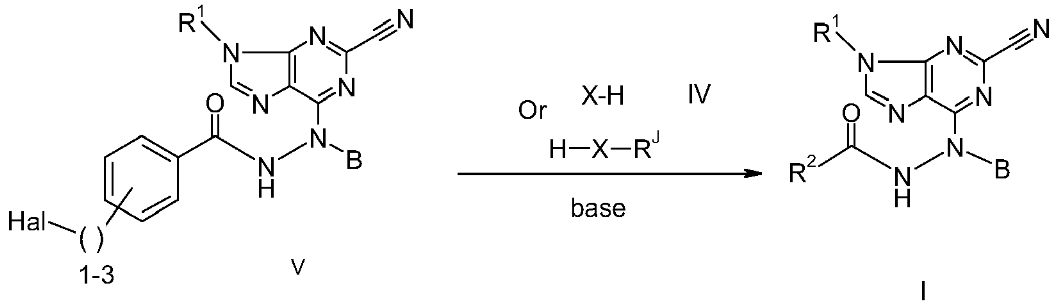


V

I

Scheme 2

Alternatively, compounds of Formula I, wherein $R^5$ is hydrogen, may be prepared from a reaction between compounds of Formula II and compounds of Formula VI, wherein X and $R^J$ are as defined for Formula I and Hal is chlorine, bromine or iodine, according to Scheme 3. Compounds II are reacted with compounds VI in the presence of a suitable base such as DIPEA or $K_2CO_3$, in a suitable solvent such as THF or ACN.

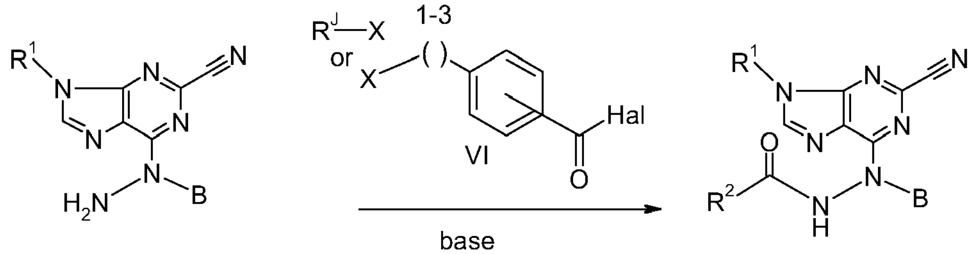


II I

Scheme 3

Compounds of Formula III are either commercially available, or they may be synthesised from the corresponding benzoic acid by reaction with a suitable reagent such as thionyl chloride (to make the acid chloride).

Compounds VI may be synthesised starting from a reaction between the corresponding methyl ester of the benzoic acid precursor of compounds of Formula III and compounds IV in the presence of a suitable base such as potassium carbonate in a suitable solvent such as DMF, followed by conversion of the methyl ester moiety to a benzoic acid moiety using a suitable reagent such as lithium hydride in a suitable solvent such as MeOH, followed by conversion to the acid halide using a suitable reagent such as thionyl chloride (to make the acid chloride), resulting in compounds VI.

Compounds of Formula V may be prepared from a reaction between compounds of Formula II and compounds of Formula III, according to Scheme 4. Compounds II are reacted with compounds III in the presence of a suitable base such as DIPEA or $K_2CO_3$, in a suitable solvent such as THF, ACN or DCM.

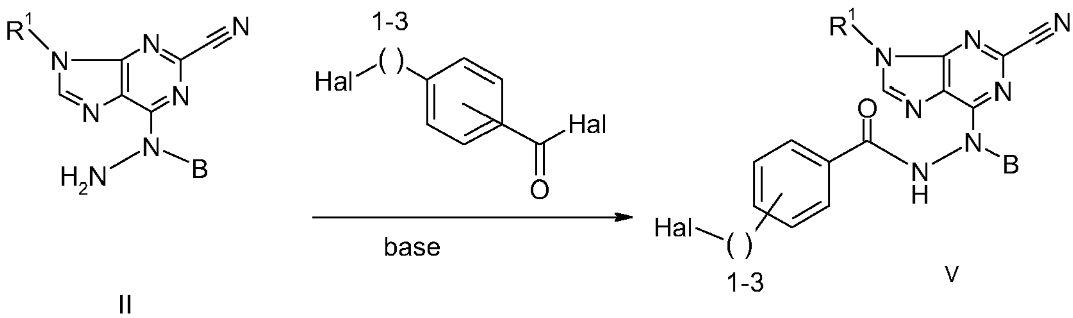


II

Scheme 4

Compounds of Formula II may be prepared from compounds of Formula VII, wherein $R^1$ and B are as defined for Formula I, according to Scheme 5 by deprotection in the presence of a suitable acid such as trifluoroacetic acid or TsOH, in a suitable solvent such as DCM or ACN.

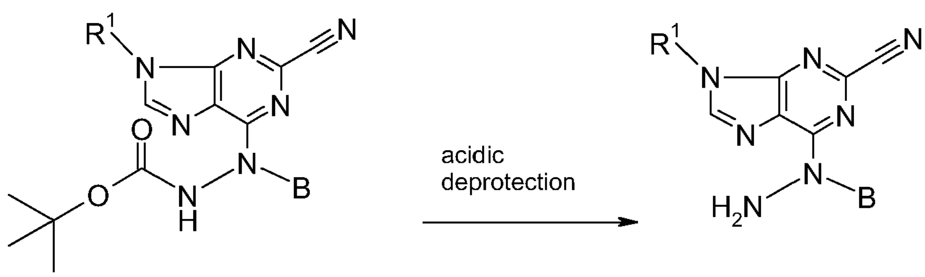


VII II

Scheme 5

Compounds of Formula VII, wherein $R^1$ represents $C_{1-4}$alkyl, may be prepared from compounds of Formula VIII, wherein $R^1$ represents $C_{1-4}$alkyl and B is as defined above for Formula I, according to Scheme 6, by cyanation, by displacement of the chloro substituent of compounds of Formula VIII using a variety of conditions, for example by treatment with potassium, sodium or zinc cyanide in the presence of a suitable base such as DABCO, or in the presence of a suitable catalyst such as $Pd_2(dba)_3/Zn$ in a suitable solvent such as DMSO or DMA, at elevated temperature, for example 100-160˚C, or in the presence of microwaves.

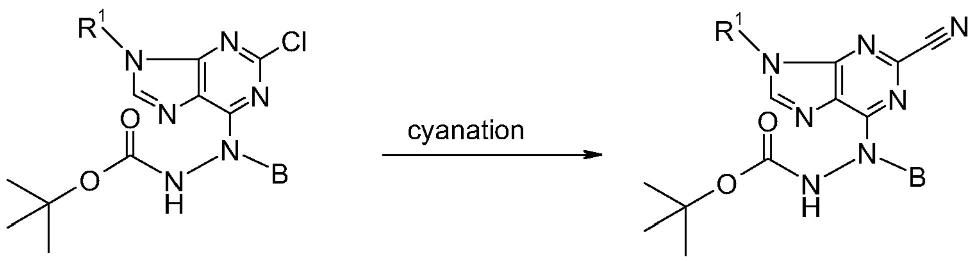


VIII VII

Scheme 6

Compounds of Formula VIII may be prepared from a reaction between compounds of Formula IX, wherein B is as defined above for Formula I, and compounds of Formula X, wherein $R^1$ represents $C_{1-4}$alkyl, according to Scheme 7. Compounds IX are reacted with compounds X in a suitable solvent such as EtOH, for example at room temperature for 3-4 days, for example according to the literature procedure given in Luo G. et al., (2002) Tetrahedron Letters, 43 (33), 5739-5742. Alternatively, compounds IX are reacted with compounds X in the presence of a suitable base such as DIPEA, in a suitable solvent, such as i-PrOH or toluene, under elevated temperature, such as 80-120˚C or in the presence of microwaves.

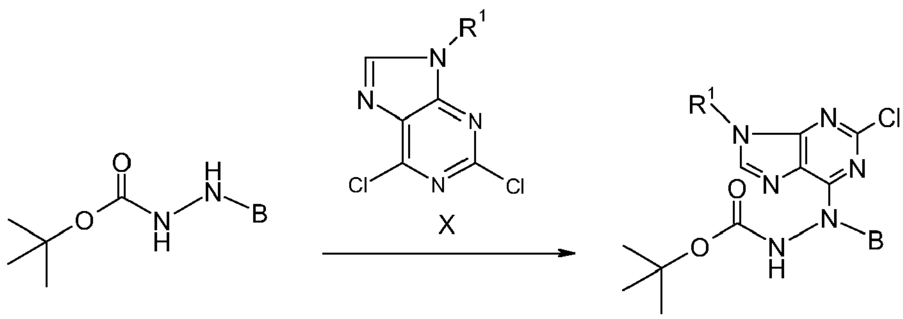

X

IX VIII

Scheme 7

Compounds of Formula IX may be prepared from the compound of Formula XI by a reductive amination reaction with a carbonyl compound XII, wherein A, n and $R^X$ are as defined for Formula I, according to Scheme 8. The compound of Formula XI, tert-butyl carbazate, is commercially available (ALDRICH). Compounds of Formula XII are also commercially available. Reductive amination of the compound XII with the compound XI is carried out in the presence of a suitable reducing agent such as hydrogen, and a suitable catalyst such as platinum or palladium or platinum oxide, or alternatively using $NaBH_3CN$ or $NaBH(AcO)_3$ in the presence of an acid such as AcOH, in a suitable solvent such as i-PrOH, EtOH, MeOH, DCE or THF, for example according to the literature procedures given in Hilpert, H. (2001) Tetrahedron, 57, 7675-7683 or Dyker, H. et al, (2001) J. Org. Chem. 66, 3760-3766).

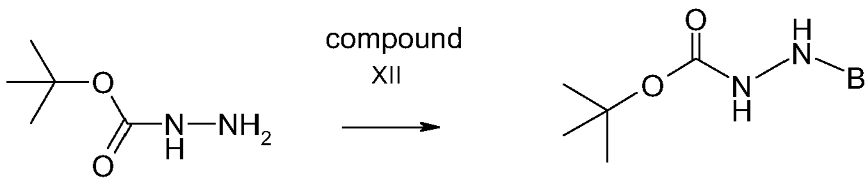


XI

IX

compound XII is 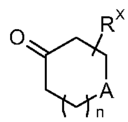 or 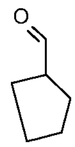 or 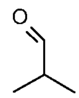

Scheme 8

Compounds of Formula I, wherein $R^1$ is $C_{1-4}$alkyl and $R^5$ is hydrogen, may altenatively be prepared from compounds of Formula XIII, wherein $R^1$ is $C_{1-4}$alkyl, $R^5$ is hydrogen and $R^2$ and B are as defined for Formula I, according to Scheme 9 by cyanation, by displacement of the chloro substituent of compounds of Formula XIII using a variety of conditions, for example by treatment with potassium, sodium or zinc cyanide in the presence of a suitable base such as DABCO, or in the presence of a suitable catalyst such as $Pd_2(dba)_3$/Zn in a suitable solvent such as DMSO or DMA, at elevated temperature, for example 100-160˚C, or in the presence of microwaves.

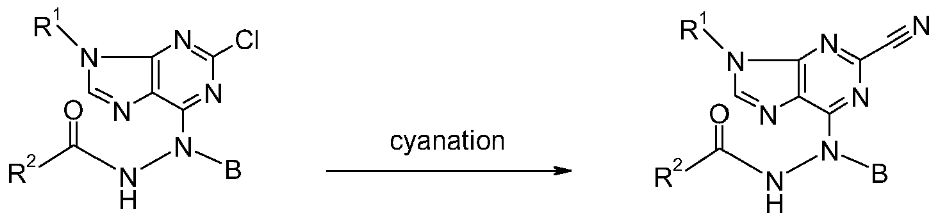


XIII

I

Scheme 9

Compounds of Formula XIII may be prepared from a reaction between compounds of Formula XIV, wherein $R^1$ is $C_{1-4}$alkyl, $R^5$ is hydrogen, B is as defined for Formula I, and Hal is chlorine, bromine or iodine, and compounds of Formula IV, according to Scheme 10. Compounds XIV are reacted with IV in the presence of a suitable base, such as DIPEA, in a suitable solvent, such as THF or ACN.

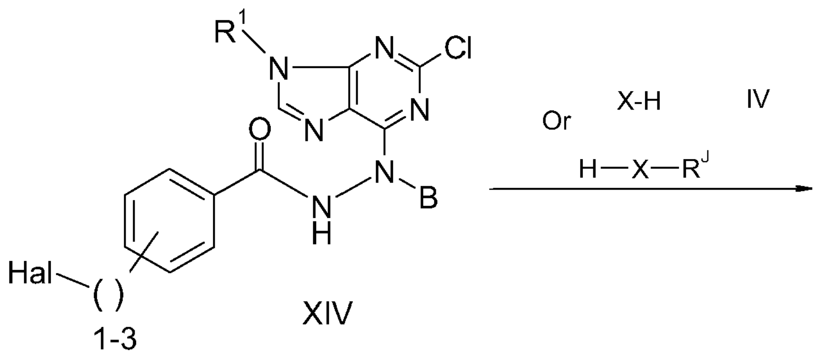


XIV

XIII

Scheme 10

Compounds of Formula XIV may be prepared according to procedures analogous to those described hereinabove for the preparation of compounds of Formula V.

Compounds of Formula VII, wherein $R^1$ represents $C_{1-4}$alkyl, $C_{1-5}$alkylene-$NR^ER^F$ or $C_{1-2}$alkylene-N-phthalimide may be prepared from compounds of Formula XV, whrerein B is as defined for Formula I, and an alkylating agent Hal-$R^1$, wherein Hal is chlorine, bromine or iodine according to Scheme 11, in the presence of a suitable base such as $K_2CO_3$, in a suitable solvent such as DMF.

If an extra equivalent of the alkylating agent Hal-$R^1$ is employed, compounds of Formula XVI may be prepared according to Scheme 11. When $R^1$ is $C_{1-5}$alkylene-$NR^ER^F$, wherein $R^E$ and $R^F$ are $C_{1-4}$alkyl, the resulting compounds of Formula XVI can be converted to compounds of Fornula I, wherein $R^1$ and $R^5$ are both $C_{1-5}$alkylene-$NR^ER^F$ and $R^E$ and $R^F$ are $C_{1-4}$alkyl, using analogous procedures to those described above in respect of compounds wherein $R^5$ is hydrogen.

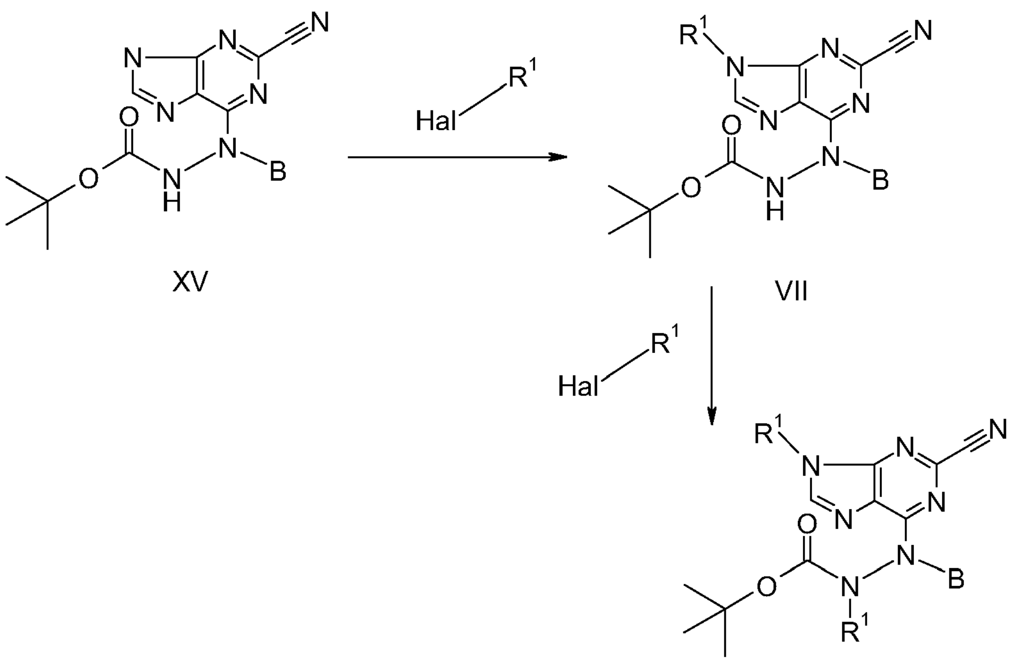

XV

VII

XVI

Scheme 11

Compounds of Formula XV may be prepared by a deprotection reaction of compounds of Formula XVII, wherein PG is a suitable protecting group such as 2-THP, and B is as defined for Formula I, according to Scheme 12. When PG is 2-THP, deprotection may be carried out in the presence of a suitable acid, for example Dowex 50 x 2 200 resin, in a

suitable solvent such as EtOH, under elevated temperature, e.g. 60-79°C.

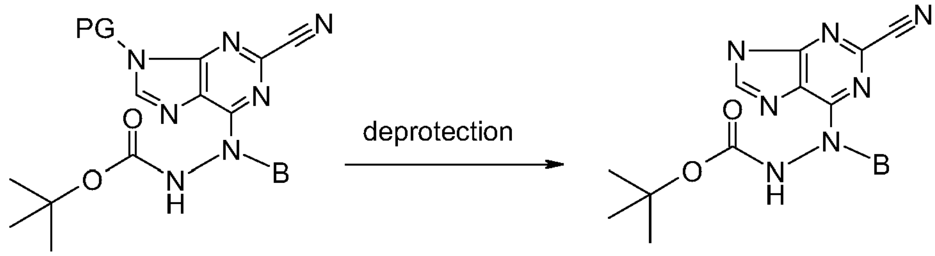


XVII XV

Scheme 12

Compounds of Formula XVII may be synthesised using analogous procedures to those described above for compounds of Formula VII.

Compounds of Fornula X, wherein $R^1$ represents $C_{1-4}$alkyl, may be prepared from the commercially available compound of Formula XVIII, by an alkylation reaction with compounds of Formula $C_{1-4}$alkyl-Hal, wherein Hal is chlorine, bromine or iodine, in a suitable solvent such as DMF or DMSO, according to Scheme 13.

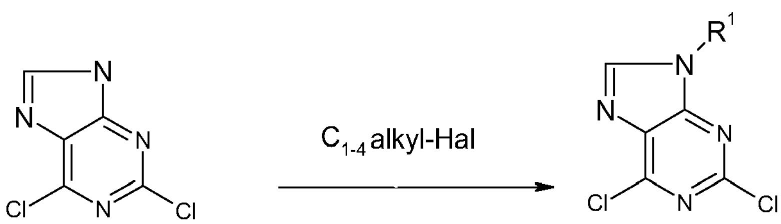


XVIII X

Scheme 13

It will be readily apparent to those skilled in the art that other compounds of Formula I may be prepared using methods analogous to those outlined above, or by reference to the experimental procedures detailed in the Examples provided herein.

Those skilled in the art will also appreciate that in the preparation of the compound of Formula I or a solvate thereof, it may be necessary and/or desirable to protect one or more sensitive groups in the molecule or the appropriate intermediate to prevent undesirable side reactions. Suitable protecting groups for use according to the present invention are well known to those skilled in the art and may be used in a conventional manner. See, for example, "Protective groups in organic synthesis" by T.W. Greene and P.G.M. Wuts (John Wiley & sons 1991) or "Protecting Groups" by P.J. Kocienski (Georg Thieme Verlag 1994). Examples of suitable amino protecting groups include acyl type protecting groups (e.g. formyl, trifluoroacetyl, acetyl), aromatic urethane type protecting groups (e.g. benzyloxycarbonyl (Cbz) and substituted Cbz), aliphatic urethane protecting groups (e.g. 9-fluorenylmethoxycarbonyl (Fmoc), t-butyloxycarbonyl (Boc), isopropyloxycarbonyl, cyclohexyloxycarbonyl) and alkyl or aralkyl type protecting groups (e.g. benzyl, trityl, chlorotrityl). Examples of suitable oxygen protecting groups may include for example alky silyl groups, such as trimethylsilyl or *tert*-butyldimethylsilyl; alkyl ethers such as tetrahydropyranyl or *tert*-butyl; or esters such as acetate.

## Examples

The following examples illustrate the invention. These examples are not intended to limit the scope of the invention, but rather to provide guidance to the skilled artisan to prepare and use the compounds, compositions, and methods of the invention. While particular embodiments of the invention are described, the skilled artisan will appreciate that various changes and modifications can be made without departing from the spirit and scope of the invention.

Intermediates

**Intermediate 1: 1,1-dimethylethyl-2-(cyclopentylmethyl)-hydrazine-carboxylate.**

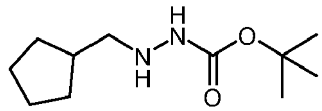

To a solution of *tert*-butyl hydrazine carboxylate (Fluka, 4.79g, 36.3mmol) in dry MeOH (80mL), cyclopentane carbaldehyde (Aldrich, 3.56mg, 36.3mmol), $NaBH_3CN$ (Aldrich, 4.55g, 72.4mmol) and glacial AcOH (8.2mL) were added. The mixture was stirred at r.t. overnight, cooled over an ice bath and neutralised with 2N NaOH solution. Solvent was evaporated and the residue was partitioned between DCM and $H_2O$. The organic layer was washed with brine, dried and concentrated to dryness. The residue was purified on silica gel (hexane/ EtOAc, 9/1) to yield the title compound. $^1H$ NMR ($CDCl_3$, 300 MHz) $\delta$: 5.96 (br s, 1 H), 3.35 (br s, 1 H), 2.77 (d, 2H, J=7.3 Hz), 2.10-1.88 (m, 1 H), 1.83-1.69 (m, 2H), 1.69-1.46 (m, 4H), 1.45 (s, 9H), 1.28-1.12 (m, 2H) ppm.

**Intermediate 2: 2,6-dichloro-9-methyl-9*H*-purine.**

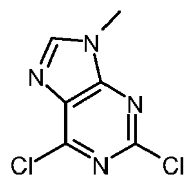

2,6-dichloro-1*H*-purine (5g, 26.5 mmol) was dissolved in DMSO (150 mL) at r.t.. Dry potassium carbonate (4g, 30.7mmol) and methyl iodide (3.29 mL, 53mmol) were added dropwise. The mixture was stirred overnight. The solution was poured into ice-water and extracted with ethyl acetate. The organic layer was dried over anhydrous sodium sulphate. The crude was purified by silica gel cartridge chromatography (Eluent: Hexane: Ethyl acetate, mixture from 100:0 to 60: 40) to give the title compound. $^1H$ NMR (300 MHz, $CDCl_3$) $\delta$ ppm: 8.09 (s, 1 H); 3.92 (s, 3H). [ES+MS] m/z 203 $(MH)^+$.

**Intermediate 3: 1,1-dimethylethyl-2-(2-chloro-9-methyl-9*H*-purin-6-yl)-2-(cyclopentyl-methyl)-hydrazine-carboxylate.**

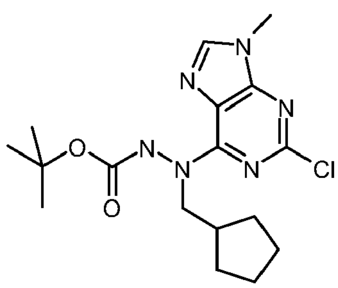

Intermediate 2 (860mg, 4.2mmol) was dissolved in i-PrOH (100mL). Intermediate 1 (1.0g, 4.6mmol) and DIPEA (1.45mL, 8.5mmol) were added and the resulting mixture was refluxed for 12 h. The reaction mixture was cooled down to r.t., the solvent was removed under reduced pressure and the crude was dissolved in DCM. The organic layer was

washed successively with sat. $NH_4Cl$, 1 N $HNaCO_3$ and brine and it was dried over anhydrous sodium sulphate. The crude mixture was purified by silica gel cartridge chromatography (Eluent: Hexane: Ethyl acetate, mixture from 100:0 to 60:30) to give the title compound. $^1H$ NMR (300 MHz, $CDCl_3$) δ ppm: 7.72 (s, 1 H); 7.40-6.62 (br., 1 H); 4.33 (br, 1H); 3.89 (br, 1H); 3.78 (s, 3H); 2.40 (m, 1H); 1.82-1.22 (m, 18H). [ES+MS] m/z 381 $(MH)^+$.

**Intermediate 4: 1,1-dimethylethyl-2-(2-cyano-9-methyl-9*H*-purin-6-yl)-2-(cyclopentylmethyl)-hydrazine-carboxylate.**

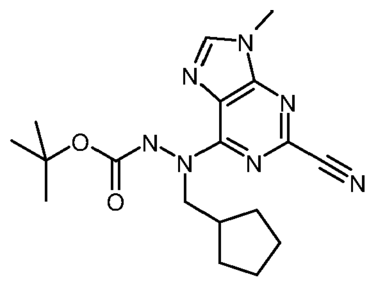

Intermediate 3 (1g, 2.63mmol), 1,1'-bis(diphenylphosphino)ferrocene (Aldrich, 20mg), Zinc (Aldrich, 58mg,), Zinc cyanide (Aldrich, 185mg, 1.58mmol) and tris-(dibenzylideneacetone)-dipalladium(0) (Aldrich, 47.6mg) were placed in a round-bottom flask under argon. DMAC (130 mL) was added under argon and the resulting solution was heated at 150˚C for 2 h. The mixture was cooled down to r.t. and filtered through Celite. After filtration, the solvent was evaporated under reduced pressure. The crude was purified by silica gel cartridge chromatography (Eluent: Hexane: Ethyl acetate, mixture from 100:0 to 60:30) to give the title compound. $^1H$ NMR (300 MHz, $CDCl_3$) δ ppm: 7.87 (s, 1 H); 7.26-6.57 (br, 1 H); 4.41 (br, 1 H); 3.93 (br, 1 H); 3.84 (s, 3H); 2.41 (m, 1 H); 1.81-1.26 (m, 18H). [ES+MS] m/z 372 $(MH)^+$.

**Intermediate 5: 6-[1-(cyclopentylmethyl)hydrazino]-9-methyl-9H-purine-2-carbonitrile.**

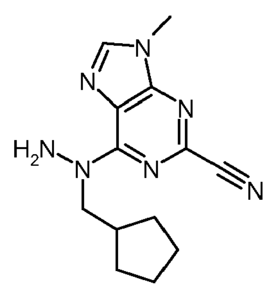

Intermediate 4 (880mg, 2.4mmol) was dissolved in DCM (20mL) and TFA (4mL) was added. The mixture was stirred at r.t. for 4 h., concentrated under reduced pressure and dissolved in DCM. The organic layer was washed with sodium bicarbonate and dried over anhydrous sodium sulphate. The crude product was purified by silica gel cartridge chromatography (Eluent: Hexane: Ethyl acetate, mixture from 100:0 to 70:30) to give the title compound. $^1H$ NMR (300 MHz, $CDCl_3$) δ ppm: 7.86 (s, 1 H); 4.22 (br, 3H); 3.84 (s,1H); 2.50 (m, 1 H); 1.80-1.22 (br, 9H). [ES+MS] m/z 272 $(MH)^+$.

**Intermediate 6: 4-(bromomethyl)-*N*'-(2-cyano-9-methyl-9*H*-purin-6-yl)-*N*'-(cyclopentylmethyl)benzohydrazide.**

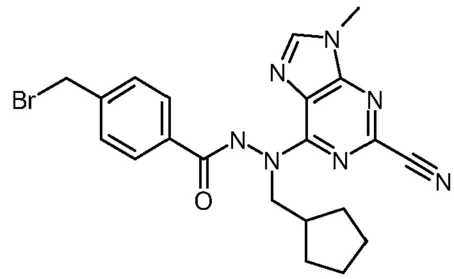

Intermediate 5 (472mg, 1.74mmol) was dissolved in ACN (60mL) at r.t.; then, dry potassium carbonate (Aldrich, 228mg, 2.09mmol) and 4-bromomethyl benzoyl bromide (Aldrich, 483mg, 1.74mmol) were added. The mixture was stirred at r.t. for 20 minutes and, then, 1N NaOH was added. The solution was concentrated under reduced pressure and the residue was extracted with AcOEt (3x15mL). The organic layers were dried over anhydrous sodium sulphate and concentrated under vacuumm. The crude was purified by silica gel cartridge chromatography (Eluent: Hexane: Ethyl acetate, mixture from 100:0 to 60:40) to give the title compound. $^1$H NMR (300 MHz, $CDCl_3$) δ ppm: 1.41-1.11 (br. 1H); 8.50-8.27 (br. 1H); 7.92 (d, 1H); 7.58 (d, 1H); 4.77 (s, 2H); 3.88 (br., 1 H); 3.80 (m,1 H); 3.73 (s, 3H); 2.34 (m, 1 H); 1.85-1.22 (m, 9H).

**Intermediate 7: 2,6-dichloro-9-(tetrahydro-2*H*-pyran-2-yl)-9*H*-purine.**

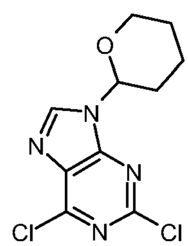

2,6-dichloropurine was dissolved in dry THF (100mL) under $N_2$ and catalytic TsOH was added (Aldrich, 62mg, 0.36mmol). The mixture was heated at 80˚C and 3,4-dihydro-2H-pyran (Aldrich 2.89mL, 32mmol) was added. The mixture was stirred at reflux for 10 h and two more days at r.t.. $NH_4OH$ (0.5mL) was added dropwise and the solvent was removed under reduced pressure. The crude was dissolved in AcOEt (40mL) and washed with sat. $NH_4Cl$ and brine. The organic layer was dried over anhydrous sodium sulphate and then, the mixture was purified by silica gel cartridge chromatography (Eluent: Hexane: Ethyl acetate, mixture from 60:40 to 0:100) to give the title compound. $^1$H NMR (300 MHz, DMSO) δ ppm: 8.94 (s, 1 H); 5.73 (m, 1 H); 4.0 (m, 1 H); 3.72 (m, 1 H); 2.23 (m, 1 H); 1.97 (m, 2H); 1.76 (m, 1 H); 1.58 (m, 2H).

**Intermediate 8: 1,1-dimethylethyl 2-[2-chloro-9-(tetrahydro-2H-pyran-2-yl)-9H-purin-6-yl]-2-(cyclopentylmethyl)hydrazinecarboxylate.**

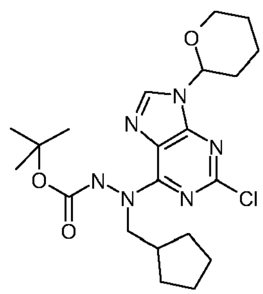

Intermediate 7 (4.9g, 18mmol) was dissolved in i-PrOH (90mL); Intermediate 1 (4.2g, 19.6mmol) and DIPEA (Fluka, 3.8mL, 21.6mmol) were added. The mixture was heated at reflux for 6 h, cooled down to r.t., the solvent was removed under reduced pressure and the crude was dissolved in DCM, extracted with sat. $NH_4Cl$, 1 N $HNaCO_3$ and brine and dried over anhydrous sodium sulphate. The crude was purified by silica gel cartridge chromatography (Eluent: Hexane: Ethyl acetate, mixture from 100:0 to 60:40) to give the title compound. $^1H$ NMR (300 MHz, DMSO) δ ppm: 9.89-9.49 (br, 1 H); 8.49-8.40 (br, 1 H); 5.59 (m, 1 H); 4.20 (m, 1 H); 3.97 (m, 1 H); 3.68 (m, 2H); 2.57 (m, 1 H); 2.26 (m, 2H); 1.93 (m, 2H); 1.69-1.16 (m, 20H). [ES+MS] m/z 451 $(MH)^+$.

**Intermediate 9: 1,1-dimethylethyl 2-[2-cyano-9-(tetrahydro-2H-pyran-2-yl)-9H-purin-6-yl]-2-(cyclopentylmethyl)hydrazinecarboxylate.**

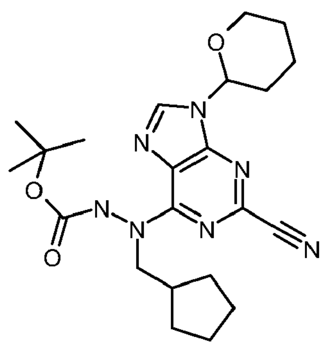

Intermediate 8 (5g, 11.08mmol), 1,1'-bis(diphenylphosphino)ferrocene (Aldrich, 244mg, 0.44mmol), Zinc (Aldrich, 87mg), Zinc cyanide (Aldrich, 775mg, 6.6mmol) and tris-(dibenzylidene-acetone)-dipalladium(0) (Aldrich, 203mg, 0.22mmol) were placed in a round-bottom flask under argon. DMAC (130mL) was added under argon and the solution was heated at 150°C for 2 h. The mixture was cooled to r.t. and filtered through Celite. After filtration, the solvent was removed under reduced pressure and the crude product was purified by silica gel cartridge chromatography (Eluent: Hexane: Ethyl acetate, mixture from 100:0 to 70:30) to give the title compound. $^1H$ NMR (300 MHz, DMSO, 80°C) δ ppm: 9.42 (br, 1 H); 8.57 (s, 1 H); 5.70 (d, 1 H); 4.28-3.87 (m, 3H); 3.74 (m, 1 H); 2.42-2.16 (m, 2H); 1.98 (m, 2H); 1.80-1.22 (m, 20H). [ES+MS] m/z 442 $(MH)^+$.

**Intermediate 10: 1,1-dimethylethyl 2-(2-cyano-1*H*-purin-6-yl)-2-(cyclo-pentylmethyl)hydrazinecarboxylate.**

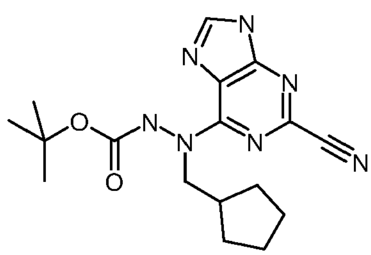

Intermediate 9 (4.4g, 9.96mmol) was dissolved in EtOH (130mL) and DOWEX 50x2-200 ion exchange resin (1.5g) was added. The solution was heated at 80°C for 1 h. The mixture was cooled down to r.t., filtered and the resin was washed with EtOH. The solvent was removed under reduced pressure and the crude mixture was purified by silica gel cartridge chromatography (Eluent: Hexane: Ethyl acetate, mixture from 100:0 to 70:30) to give the title compound. $^1H$ NMR (300 MHz, DMSO 25°C) δ ppm: 9.89-9.50 (br, 1 H); 8.45 (s, 1 H); 4.30 (br, 1 H); 3.70 (br, 1 H); 2.29 (m, 1 H); 1.73-1.24 (m, 17H). [ES+MS] m/z 358 $(MH)^+$.

**Intermediate 11: 1,1-dimethylethyl 2-cyclopentylhydrazinecarboxylate.**

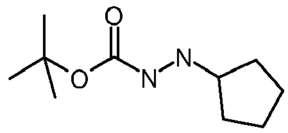

A solution of cyclopentanone (Aldrich, 4 mL, 45mmol) in MeOH (160ml) was treated with tert-butyl carbazate (Fluka, 5.9g, 45mmol), $NaBH_3CN$ (Aldrich, 4.24g, 67.5mmol) and glacial AcOH (10mL). The mixture was stirred at r.t. overnight, cooled over an ice bath and neutralised with 2N NaOH solution. The solvent was evaporated and the residue was partitioned between DCM and $H_2O$. The organic phase was washed with brine, dried and the solvent evaporated under reduced pressure to give the title compound. $^1H$ NMR (300 MHz, DMSO-d6) δ ppm: 8.20 (br.s, 1 H); 4.09 (br.s, 1 H); 1.30-1.59 (m, 8H); 1.37 (s, 9H).

**Intermediate 12: 1,1-dimethylethyl 2-[2-chloro-9-(tetrahydro-2*H*-pyran-2-yl)-9*H*-purin-6-yl]-2-cyclopentylhydrazinecarboxylate.**

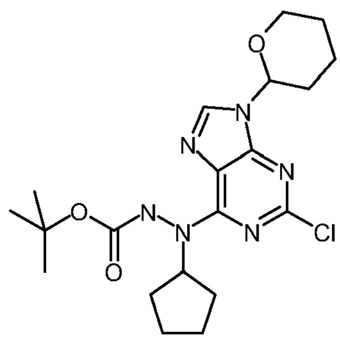

A mixture of Intermediate 11 (2.4g, 12mmol), Intermediate 7 (3.28g, 12mmol) and DIPEA (Fluka, 4.1ml, 24mmol) in dry i-PrOH (43ml) was stirred under reflux for 15 h. The mixture was concentrated under reduced pressure and the residue partitioned between DCM and 1M ammonium chloride. The organic layer was treated with brine and dried over $MgSO_4$. The residue was purified by flash chromatography (eluent: Hex/AcOEt mixtures 98:2 to 50:50) to give the title compound. [ES+ MS] m/z 437 $(MH)^+$.

**Intermediate 13: 1,1-dimethylethyl 2-[2-cyano-9-(tetrahydro-2*H*-pyran-2-yl)-9*H*-purin-6-yl]-2-cyclopentylhydrazinecarboxylate.**

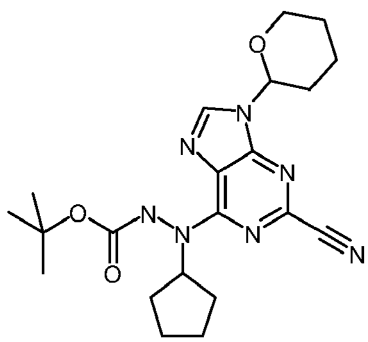

A mixture of Intermediate 12 (210mg, 0.48mmol), $Zn(CN)_2$ (Aldrich, 34mg, 0.29mmol), $Pd_2(dba)_3$ (Aldrich, 9mg, 0.01mmol), dppf (Aldrich, 11mg, 0.02mmol), and Zn powder (Aldrich, 4mg, 0.06mmol) were placed in a microwave flask flushed with argon. DMA (Aldrich, 4mL) was added via syringe. The resulting mixture was heated in the microwave at 150˚C for 180s and 12bar of pressure. The mixture was cooled down to r.t., filtered through Celite, concentrated under vacuumm and the residue was purified by flash chromatography (eluent: Hex/AcOEt mixtures 95:5 to 50:50) to give the title compound. [ES+MS] m/z 428 $(MH)^+$.

**Intermediate 14: 1,1-dimethylethyl 2-(2-cyano-1*H*-purin-6-yl)-2-cyclo-pentylhydrazine-carboxylate.**

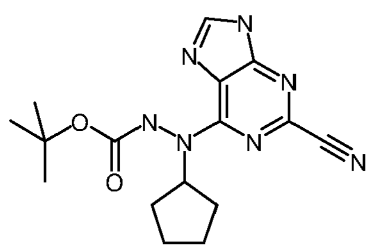

A mixture of Intermediate 13 (2.76g, 6.5mmol) and Dowex D-50 $(H^+)$ (Aldrich, 1.1g) in EtOH (150mL) was refluxed until the starting material was consumed (1 h., followed by HPLC). The mixture was cooled, filtered and the residue was washed with hot EtOH. The combined organic fractions were concentrated under vacuumm and the residue was purified by flash chromatography (eluent: Hex/AcOEt mixtures 98:2 to 0:100) to give the title compound. [ES+MS] m/z 344 $(MH)^+$.

**Intermediate 15: 1,1-dimethylethyl 2-(2-cyano-9-methyl-9*H*-purin-6-yl)-2-cyclopentyl-hydrazine-carboxylate.**

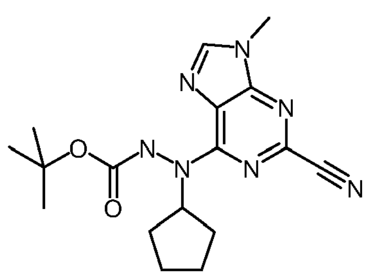

To a solution of Intermediate 14 (955mg, 2.78mmol) in DMF (25mL) at r.t., anhydrous potassium carbonate (Aldrich, 461 mg, 3.34mmol) and methyl iodide (Aldrich, 0.26mL, 4.17mmol) were added at 0˚ C. The resulting mixture was stirred at 0˚ C under $N_2$ for 2 h and the residue was partitioned between AcOEt and water and the aqueous layer

was extracted three times with AcOEt. The organic layer was treated with brine and dried over $MgSO_4$. The residue was purified by flash chromatography (eluent: Hex/AcOEt mixtures 98:2 to 50:50) to give the title compound. [ES+MS] m/z 358 $(MH)^+$.

**Intermediate 16: 6-(1-cyclopentylhydrazino)-9-methyl-9*H*-purine-2-carbonitrile.**

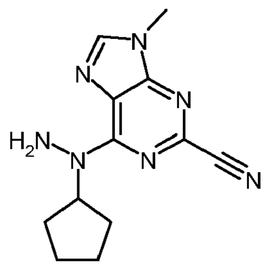

To a solution of Intermediate 15 (750mg, 2.1 mmol) in ACN (16mL), TsOH (Aldrich, 904mg, 5.25mmol) was added and the resulting reaction mixture was stirred at r.t. overnight. The mixture was concentrated under vacuumm and the residue partitioned between DCM and water. The organic layer was treated with a saturated solution of sodium bicarbonate, brine and dried over anhydrous $Na_2SO_4$. The residue was purified by flash chromatography (eluent: Hex/ AcOEt mixtures 98:2 to 30:70) to give the title compound. [ES+MS] m/z 258 $(MH)^+$.

**Intermediate 17: 4-(bromomethyl)-*N'*-(2-cyano-9-methyl-9*H*-purin-6-yl)-*N'*-cyclopentyl-benzohydrazide.**

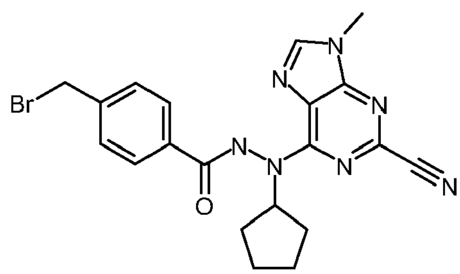

To a solution of Intermediate 16 (458mg, 1.78mmol) in THF (16mL), DIPEA (Fluka, 755μl, 4.45mmol) and 4-bromomethyl benzoyl bromide (Aldrich, 495mg, 1.78mmol) were added. The resulting reaction mixture was stirred overnight at r.t. under $N_2$. The mixture was concentrated under reduced pressure and the residue partitioned between DCM and water; the organic layer was treated with 1 N NaOH, brine and dried over anhydrous $Mg_2SO_4$. The residue was purified by flash chromatography (eluent: Hex/AcOEt mixtures 95:5 to 30:70) to give the title compound. [ES+MS] m/z 454 $(MH)^+$.

**Intermediate 18: 1-(Diphenylmethyl)-3-azetidinyl methanesulfonate.**

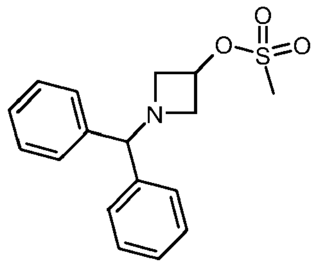

Under a nitrogen atmosphere, TEA (0.9mL, 6.5mmol) was added to a solution of 1-benzhydrylazetan-3-ol (Maybridge, 1.03g, 4.3mmol) in dry DCM (15mL), previously cooled to 0 ˚C. After 5 min, methanesulfonyl chloride (Aldrich, 0.4mL, 5.2mmol) was added and the resulting reaction mixture was stirred at 0 ˚C for 2 h. Water was then added and the mixture was extracted with DCM. The combined organic layers were dried over anhydrous $MgSO_4$ and solvent was removed *in* vacuo to yield the title compound. $^1H$ NMR (300 MHz, DMSO-$d_6$) δ ppm: 7.58- 7.10 (br., 10H); 5.19- 5.03 (br., 1 H); 4.60- 4.42 (br., 1 H); 3.63- 3.42 (br., 2H); 3.17 (br.s, 3H); 3.15- 2.97 (br., 2H).

**Intermediate 19: 1-[1-(Diphenylmethyl)-3-azetidinyl]pyrrolidine.**

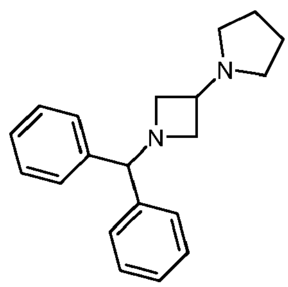

Intermediate 18 (0.5g, 1.6mmol) was dissolved in pyrrolidine (Aldrich, 3 mL, 36mmol) and the resulting reaction mixture was heated to 60 ˚C for 2 h, then, to 70 ˚C for further 2h and, finally, refluxed for 8 h, before being cooled down to r.t. overnight. A saturated aqueous solution of sodium bicarbonate was added and the product was extracted with DCM. The combined organic layers were dried over anhydrous $Na_2SO_4$ and solvent was evaporated under reduced pressure to yield the title compound. $^1H$ NMR (300 MHz, DMSO-$d_6$) δ ppm: 7.45- 7.36 (m, 4H); 7.31- 7.11 (m, 6H); 4.39 (s, 1 H); 3.55- 3.46 (m, 1 H); 3.13- 3.04 (m, 1 H); 3.04- 2.92 (m, 1 H); 2.84- 2.76 (m, 2H); 2.37- 2.23 (br., 4H); 1.70-1.56 (br., 4H). [ES+MS] m/z 293 $(MH)^+$.

**Intermediate 20: 1-(3-Azetidinyl)pyrrolidine dihydrochloride.**

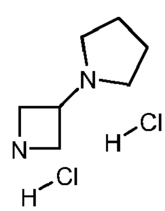

To a solution of Intermediate 19 (1.6mmol) in EtOH (5.2mL), 1N HCl (1.4mL) and palladium hydroxide on carbon (Aldrich, 0.05mg, 0.38mmol) were added and the resulting reaction mixture was hydrogenated at r.t. and 3bar over the weekend. More 1N HCl (3mL) was added and the hydrogenation was kept for further 4 days. The reaction mixture was then lyophilized and, then, MeOH was added. The resulting solution was washed with EtOAc, hexane, DCM and diethyl ether to yield the title compound which was used in subsequent steps without any further purification. [ES+

MS] m/z 127 $(MH)^+$.

**Intermediate 21: 1,1-dimethylethyl 2-cyclohexylhydrazinecarboxylate.**

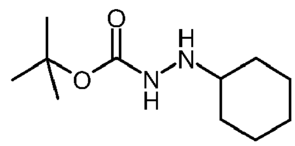

This Intermediate was prepared using a method analogous to the one described for Intermediate 11 but replacing cyclopentanone with cyclohexanone. [ES+MS] m/z 215 $(MH)^+$.

**Intermediate 22: 1,1-dimethylethyl 2-(2-chloro-9-methyl-9*H*-purin-6-yl)-2-cyclohexyl-hydrazine-carboxylate.**

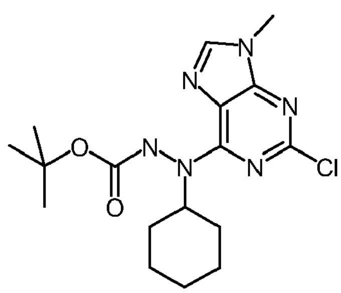

A mixture of Intermediate 21 (1.55g, 7.25mmol), Intermediate 2 (1.62g, 7.98mmol), DIPEA (Fluka, 2.5ml, 14.5mmol) and dry i-PrOH (26ml) was stirred under reflux for 48 h. The mixture was concentrated under reduced pressure and the residue partitioned between DCM and 1M ammonium chloride. The organic layer was treated with brine and dried over $MgSO_4$. The residue was purified by flash chromatography (eluent: Hex/AcOEt mixtures 95:5 to 30:70) to give the title compound. [ES+MS] m/z 381 $(MH)^+$.

**Intermediate 23: 1,1-dimethylethyl 2-(2-cyano-9-methyl-9*H*-purin-6-yl)-2-cyclohexyl-hydrazine-carboxylate.**

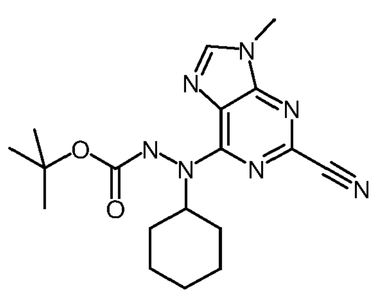

A mixture of Intermediate 22 (400mg, 1.05mmol), $Zn(CN)_2$ (Aldrich, 74mg, 0.63mmol), $Pd_2(dba)_3$ (Aldrich, 19mg, 0.02mmol), dppf (Aldrich, 23mg, 0.04mmol), and Zn powder (Aldrich, 9mg, 0.13mmol) were placed in a microwave flask which was flushed with argon. DMA (Aldrich, 5mL) was added via syringe. The resulting mixture was heated in the microwave at 150˚C for 300 seconds and 12 bar of pressure. The mixture was cooled down to r.t., filtered through Celite and washed with DCM. The solution was concentrated under vacuumm and the residue was purified by flash chromatography (eluent: Hex/AcOEt mixtures 98:2 to 0:100) to give the title compound. [ES+MS] m/z 372 $(MH)^+$.

**Intermediate 24: 6-(1-cyclohexylhydrazino)-9-methyl-9H-purine-2-carbonitrile.**

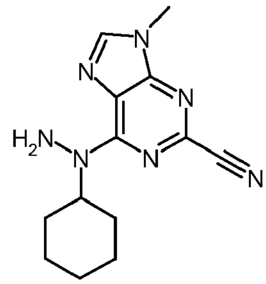

To a solution of Intermediate 23 (1.36g, 3.7mmol) in DCM (32mL), TsOH (Aldrich, 1.6g, 9.25mmol) was added and the resulting reaction mixture was stirred at r.t. overnight. The mixture was concentrated under reduced pressure and the residue partitioned between DCM and water and the organic layer was treated with a saturated solution of sodium bicarbonate, brine and dried over anhydrous $Na_2SO_4$ to give the title compound. The residue was purified by flash chromatography (eluent: Hex/AcOEt mixtures 92:8 to 20:80) to give the title compound. [ES+MS] m/z 272 $(MH)^+$.

**Intermediate 25: 4-(bromomethyl)-*N'*-(2-cyano-9-methyl-9*H*-purin-6-yl)-*N'*-cyclohexyl-benzohydrazide.**

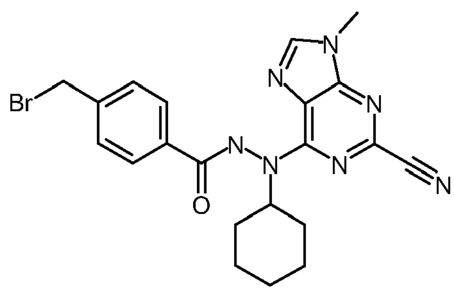

To a solution of Intermediate 24 (865mg, 3.19mmol) in THF (25mL), DIPEA (Fluka, 1.4mL, 7.96mmol) and 4-bromomethyl benzoyl bromide (Aldrich, 887mg, 3.19mmol) were added. The resulting reaction mixture was stirred at r.t. and under $N_2$ overnight. The mixture was concentrated under reduced pressure and the residue partitioned between DCM and water and the organic layer was treated with 1 N NaOH, brine and dried over anhydrous $Mg_2SO_4$. The residue was purified by flash chromatography (eluent: Hex/AcOEt mixtures 95:5 to 30:70) to give the title compound. [ES+MS] m/z 468 $(MH)^+$.

**Intermediate 26: 1,1-dimethylethyl 2-{2-cyano-9-[3-(dimethylamino)propyl]-9H-purin-6-yl}-2-(cyclopentylmethyl)hydrazinecarboxylate trifluoroacetate.**

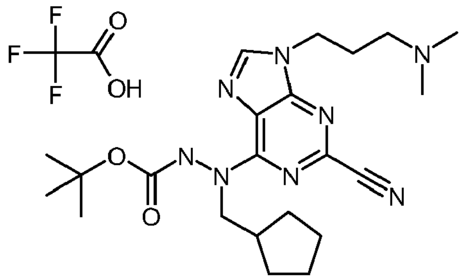

Intermediate 10 (50mg., 0.14mmol) was dissolved in anhydrous DMF (5mL) under nitrogen. Anhydrous potassium carbonate (39mg, 0.28mmol) and 3-Dimethyl-aminopropyl chloride hydrochloride (Aldrich, 30mg, 0.21 mmol) were added and the mixture was stirred at 50˚C overnight. The solution was filtered and concentrated under reduced pressure. The crude was dissolved in DCM and washed successively with sat. $NH_4Cl$ and brine. The organic layer was dried over anhydrous sodium sulphate, concentrated to dryness and the residue was purified by preparative HPLC (SUNFIRE 19x150 mm, ACN: H2O, 0.1%TFA, gradient 10-100%) to give the title compound. $^1H$ NMR (300 MHz, DMSO, 80˚C) δ ppm: 9.34 (br, 1 H); 8.43 (s, 1 H); 4.29 (t, 2H); 3.12 (m, 2H); 2.78 (s, 6H); 2.35 (m, 1 H); 2.25 (m, 2H); 2.0-1.15 (m, 17H). [ES+MS] m/z 443 $(MH)^+$.

**Intermediate 27: 1,1-dimethylethyl 2-{2-cyano-9-[2-(dimethylamino)ethyl]-9H-purin-6-yl}-2-(cyclopentylmethyl) hydrazinecarboxylate trifluoroacetate.**

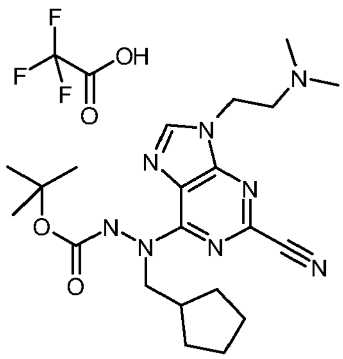

Intermediate 10 (50mg., 0.14mmol) was dissolved in anhydrous DMF (5mL) under $N_2$. Anhydrous potassium carbonate (Aldrich, 39mg., 0.28mmol) and 2-dimethylaminoethyl chloride hydrochloride (Aldrich, 30mg, 0.21 mmol) were added and the mixture was stirred at 50˚C overnight. The solution was then filtered and concentrated under reduced pressure. The crude was dissolved in DCM and washed successively with sat. $NH_4Cl$ and brine. The organic layer was dried over anhydrous sodium sulphate, concentrated to dryness and the residue was purified by preparative HPLC (SUNFIRE 19x150 mm, ACN: H2O, 0.1%TFA, gradient 10-100%) to give the title compound. $^1H$ NMR (300 MHz, DMSO, 80˚C) δ ppm: 7.98 (d, 2H); 7.53 (d, 2H); 7.76-7.61 (br, 1 H); 4.51 (br, 1 H); 4.20 (s, 2H); 4.01 (br, 1 H); 3.78 (s, 3H); 3.50 (br, 2H); 3.36 (m, 1 H); 3.18 (br, 3H); 2.88 (br, 3H); 2.77 (s, 3H); 2.37 (m, 1 H); 2.00 -1.21 (m, 13H). [ES+MS] m/z 429 $(MH)^+$.

**Intermediate 28: 6-[1-(cyclopentylmethyl)hydrazino]-9-[2-(dimethylamino)ethyl]-9*H*-purine-2-carbonitrile trifluoroacetate.**

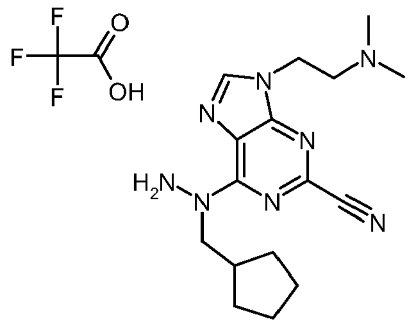

Intermediate 27 (300mg., 0.7mmol) was dissolved in DCM (30mL) under $N_2$ and TFA (2.5mL) was added. The mixture was stirred at r.t. overnight. The solvent was removed under reduce pressure and the resulting oil was used in the next step without further purification step.

**Intermediate 29: 6-(1-cyclopentylhydrazino)-9-[3-(dimethylamino)propyl]-9*H*-purine-2-carbonitrile trifluoroacetate.**

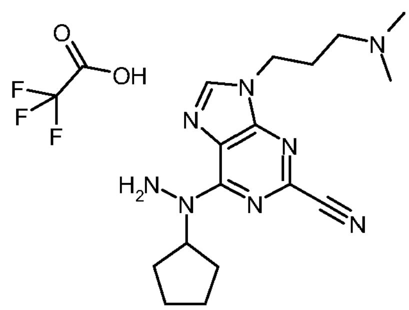

Intermediate 26 (100mg, 0.23mmol) was dissolved in DCM (10mL) under $N_2$ and TFA (Fluka, 1 mL) was added. The mixture was stirred at r.t overnight. The solvent was removed under reduced pressure and the crude product was purified by preparative HPLC (SUNFIRE 19x150 mm, ACN: H2O, 0.1%TFA, gradient 10-100%) to give the title compound. $^1H$ NMR (300 MHz, DMSO+$D_2O$) δ ppm: 8.37 (s, 1 H); 5.68 (br, 1 H); 4.24 (t, 2H); 3.04 (m, 2H); 2.73 (s, 6H); 2.16 (m, 2H); 1.91-1.43 (m, 8H).

**Intermediate 30: 1,1-Dimethylethyl-2-(2-methylpropyl)hydrazinecarboxylate**

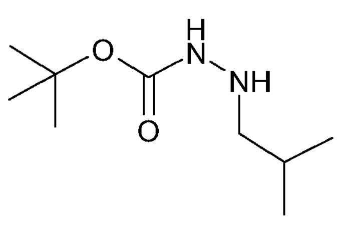

A solution of 1,1-dimethylethyl hydrazinecarboxylate (ALDRICH, 9.2 g, 70 mmol) in i-PrOH (50 ml) was treated at 0˚C with *i*-butylaldehyde (6.4 ml, 70 mmol) over 15 min and stirring at 0˚C for 2 h, then the mixture was stirred 5 h at room temperature. To this solution containing the intermediate hydrazone was added $PtO_2$ and the suspension was hydrogenated at room temperature and 2.6 bar for 48 h. The suspension was filtered and the solvent was removed under reduced pressure to give the title compound. $^1H$ NMR (300 MHz, $CDCl_3$) δ ppm: 6.02 (br.s, 1 H), 3.92 (br.s, 1 H), 2.66 (d, 2H), 1.73 (m, 1 H), 1.46 (s , 9H), 0.93 (d, 6H) [ES+ MS] m/z 189 ($MH^+$).

**Intermediate 31: 1,1-Dimethylethyl 2-(2,2-dimethylpropyl)hydrazinecarboxylate**

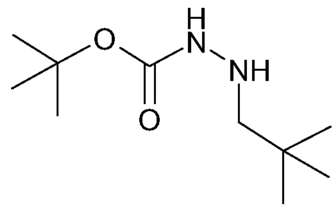

The title compound was prepared by a method analogous to that described above for Intermediate 30, replacing *i*-butylaldehyde with trimethylacetaldehyde. $^1H$ NMR (300 MHz, $CDCl_3$) δ ppm: 8.19 (s, 1 H), 3.34 (br.s, 1 H), 2.46 (d, 2H), 1.37 (s, 9H), 0.85 (s, 9H) [ES+ MS] m/z 203 ($MH^+$).

**Intermediate 32: 1,1-Dimethylethyl-2-(2-chloro-4-pyrimidinyl)-2-(2-methylpropyl)-hydrazinecarboxylate**

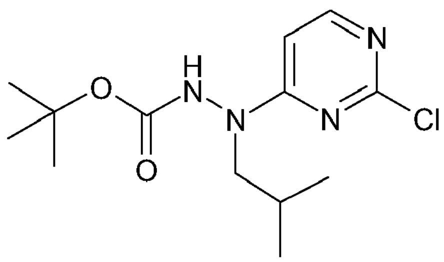

A mixture of Intermediate 30 (12.07 g, 64 mmol), 2,4-dichloropyrimidine (19.07 g, 128 mmol), triethylamine (10.7 ml, 145 mmol) and dry EtOH (100 ml) was stirred at room temperature for 3 days. The mixture was concentrated under reduced pressure and the residue partitioned between DCM and 1M ammonium chloride. The organic layer was treated with brine and dried over $MgSO_4$. The residue was purified by flash chromatography (eluent: Hex/AcOEt mixtures 97:3 to 50:50) to give the title compound. $^1H$ NMR (300 MHz, DMSO-d6) δ ppm: 9.77 (br.s, 1 H), 8.15 (d, 1 H), 6.52 (br.s, 1 H), 3.76 (br.s, 1 H), 3.17 (br.s, 1 H), 1.92 (m, 1 H), 1.43 (s, 9H), 0.87 (m, 6H). [ES- MS] m/z 299 ($MH^-$).

**Intermediate 33: 1,1-Dimethylethyl-2-(2-chloro-4-pyrimidinyl)-2-(2,2-dimethylpropyl)-hydrazinecarboxylate**

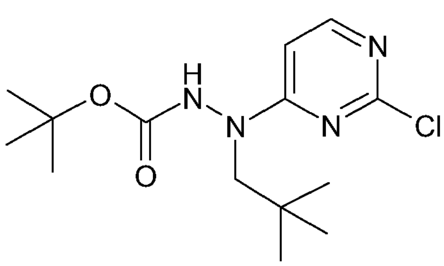

The title compound was prepared by a method analogous to that described above for Intermediate 32, replacing Intermediate 30 with Intermediate 32. $^1H$ NMR (300 MHz, $CDCl_3$) δ ppm: 8.12 (d, 1 H), 6.64 (br.s, 1 H), 6.58 (d, 1 H), 1.50 (s, 9H), 1.00 (s, 9H). [ES- MS] m/z 313 ($MH^-$).

**Intermediate 34: 1,1-Dimethylethyl-2-(2-cyano-4-pyrimidinyl)-2-(2-methylpropyl)-hydrazinecarboxylate**

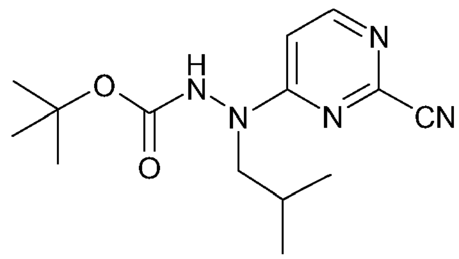

Potassium cyanide (3.5 g, 54.6 mmol) was added to a suspension of Intermediate 32 (13.69 g, 45.51 mmol) and DABCO (5.10 g, 45.51 mmol) in a mixture of DMSO/$H_2O$ 85/15 (195 ml) at room temperature. The reaction mixture was stirred at 100 ˚C for 1.7 h, poured into ice water (250 ml). The white solid was filtered off and dried. The compound was purified by flash chromatography (eluent: Hex/AcOEt mixtures 4:1 to 3:1) to give the title compound. $^1$H NMR (300 MHz, DMSO-d6) δ ppm: 9.88 (br.s, 1 H), 8.37 (d, 1 H), 6.80 (br.s, 1 H), 3.60 (br.s, 1 H), 3.25 (br.s, 1 H), 1.95 (m, 1 H), 1.43 (s, 9H), 0.88 (m, 6H). [ES- MS] m/z 290 ($MH^-$).

**Intermediate 35: 1,1-Dimethylethyl-2-(2-cyano-4-pyrimidinyl)-2-(2,2-dimethylpropyl)-hydrazinecarboxylate**

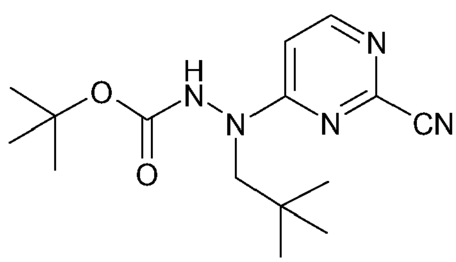

The title compound was prepared prepared by a method analogous to that described above for Intermediate 34, replacing Intermediate 32 with Intermediate 33. $^1$H NMR (300 MHz, DMSO-d6): 9.89 (br.s, 1 H), 8.39 (d, 1 H), 6.79 (br.s, 1 h), 3.96 (d, 1 H), 3.10 (d, 1 H), 1.43 (s, 9H), 0.93 (s, 9H). [ES+ MS] m/z 306 ($MH^+$).

**Intermediate 36: 4-[1-(2-Methylpropyl)hydrazino]-2-pyrimidinecarbonitrile**

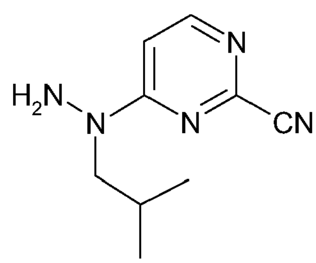

Intermediate 34 (8.2 g, 28.15 mmol) was dissolved in a mixture of TFA (12 ml) in DCM (120 ml). The reaction mixture was stirred at room temperature overnight. More TFA (5 ml) was added and after 2 h the reaction was finished. The mixture was treated with water (2 x 100 ml), 10% $NaHCO_3$ (100 ml), dried over $Na_2SO_4$ and evaporated under reduced pressure to give the title compound. $^1$H NMR (300 MHz, DMSO-d6): 8.17 (d, 1 H), 7.23 (d, 1 H), 4.93 (s, 2H),

3.52 (d, 1 H), 2.17 (m, 1 H), 0.84 (d, 6H). [ES+ MS] m/z 292 ($MH^+$).

**Intermediate 37: 4-[1-(2,2-Dimethylpropyl)hydrazino]-2-pyrimidinecarbonitrile**

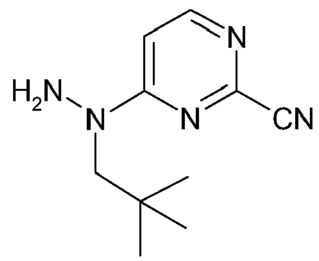

The title compound was prepared by a method analogous to that described above for Intermediate 36, replacing Intermediate 34 with Intermediate 35. $^1$H NMR (300 MHz, DMSO-d6): 8.19 (d, 1 H), 7.22 (d, 1 H), 4.99 (s, 2H), 3.55 (s, 2H), 0.94 (s, 9H). [ES+ MS] m/z 206 $(MH)^+$

**Intermediate 38: 4-[(4-Methyl-1-piperazinyl)methyl]benzoic acid**

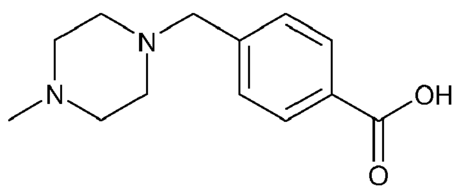

A solution of N-methylpiperazine (ALDRICH, 1.46 ml, 13.1 mmol) in dimethylformamide (5 ml) was cooled to 0° C and, then, potassium carbonate ($K_2CO_3$, 1.81 g, 13.1 mmol) was added. This mixture was stirred at 0° C for 30 min. Then, methyl 4-(bromomethyl) benzoate (ALDRICH, 3 g, 13.1 mmol) was added. The reaction mixture was allowed to warm up to room temperature and stirred for 17 h. The mixture was concentrated under reduce pressure. The residue was dissolved in DCM and washed with water, the aqueous layer was extracted with DCM. The organic layers were combined, washed with water, dried over $MgSO_4$ filtered and the solvent removed under reduce pressure to give the corresponding methyl 4-[(4-methyl-1-piperazinyl)methyl]benzoate. $^1$H RMN (300 MHz, CDCl3-d6): 7.97 (d, 2H), 7 40 (d, 2H), 3.90 (s, 3H), 3.55 (s, 2H), 2.47 (br. m, 8H), 2.28 (s, 3H).

A solution of lithium hydroxide (ALDRICH, 337 mg, 14.1 mmol) in $H_2O$ (10 ml) was added to a solution of the methyl ester (1.4 g, 5.63 mmol) in MeOH (20 ml) and the mixture was heated at reflux for 2h. The mixture was concentrated under reduced pressure. The residue was dissolved in DCM and 2N hydrochloric acid was added to give pH 5. The aqueous layer was partitioned with n-Butanol (5 times) and the fractions were combined, dried over $MgSO_4$, filtered and evaporated under reduce pressure to give the title compound as a white solid. $^1$H RMN (300 MHz, DMSO-d6): 12.83 (br. m, 1 H), 11.05 (br. m, 1 H), 7.90 (d, 2H), 7.43 (d, 2H), 4.36 (m, 1 H), 3.60 (s, 2H), 3.38-2.80 (br. m, 8H), 2.68 (s, 3H). [ES+ MS] m/z 235 $(MH)^+$.

**Intermediate 39: 1,1-dimethylethyl 2-(5-bromo-2-chloro-4-pyrimidinyl)-2-(2-methyl propyl)hydrazinecarboxylate.**

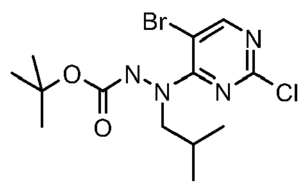

To a solution of 5-bromo-2,4-dichloropyrimidine (ALDRICH, 3.0 g, 13 mmol) and Intermediate 30 (2.5 g, 13 mmol) in *i*-PrOH (80 mL), N,N-diisopropylethylamine (3 mL, 17 mmol) was added and the resulting reaction mixture was refluxed for 5 h. The mixture was concentrated under reduced pressure and the residue partitioned between DCM and 1M ammonium chloride. The organic layer was washed with water and brine and dried over anhydrous $Na_2SO_4$. The residue was purified by flash chromatography (eluant: Hex/EtOAc 9:1) to give the title compound. $^1H$ NMR (300 MHz, $CDCl_3$) $\delta$ ppm: 8.27 (br.s, 1 H), 6.85-6.40 (br.m, 1 H), 4.15-3.00 (br.m, 2H), 2.06 (m, 1 H), 1.55-1.30 (br.m, 9H), 0.97 (d, 6H) [ES+ MS] m/z 379 $(M)^+$, [ES- MS] m/z 377 $(M-2H)^-$.

**Intermediate 40: 1,1-dimethylethyl 2-(5-bromo-2-cyano-4-pyrimidinyl)-2-(2-methyl propyl)hydrazinecarboxylate.**

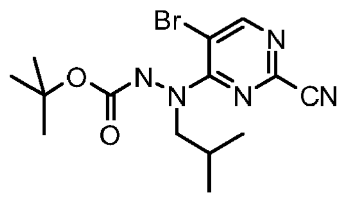

Potassium cyanide (0.9 g, 13 mmol) was added to a suspension of Intermediate 39 (4.1 g, 11 mmol) and DABCO (1.2 g, 11 mmol) in a mixture of $DMSO/H_2O$ 9:1 at room temperature. The reaction mixture was stirred at room temperature for 3 h, and then, poured into iced water. The cream colour solid that precipitated was filtered off, washed abundantly with water and dried under air. The compound was purified by flash chromatography (eluant: Hex/EtOAC 19:1) to give the title compound. $^1H$ NMR (300 MHz, $CDCl_3$) $\delta$ ppm: 8.46 (br.s, 1 H), 6.85-6.45 (br.m, 1 H), 4.15-3.0 (br.m, 2H), 2.06 (m, 1 H), 1.52-1.31 (br.m, 9H), 0.97 (d, 6H); $^1H$ NMR (300 MHz, $d_6$-DMSO, 80°C) $\delta$ ppm: 9.77 (br.s, 1 H), 8.59 (s, 1 H), 3.59 (br.s, 2H), 2.04 (m, 1 H), 1.41 (br.s, 9H), 0.92 (d, 6H); $^{13}C$ NMR (90 MHz, $d_6$-DMSO) $\delta$ ppm: 160.7, 159.6, 153.6, 140.6, 115.7, 104.7, 80.4, 58.6, 27.7, 25.5, 20.0. [ES+ MS] m/z 370 $(M)^+$.

**Intermediate 41: 5-bromo-4-[1-(2-methylpropyl)hydrazino]-2-pyrimidine carbonitrile.**

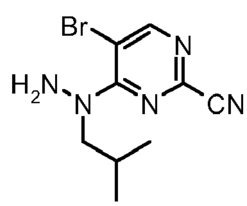

To a solution of Intermediate 40 (3.5 g, 9.4 mmol) in dry acetonitrile (70 mL), 4-toluenesulfonic acid (4.07 g, 23.6 mmol) was added and the resulting reaction mixture was stirred at room temperature for 24 h. The mixture was then concentrated *in vacuo* and the residue partitioned between DCM and sat. sodium bicarbonate. The residue was purified by preparative HPLC (X-TERRA 19x150 mm, $ACN:H_2O$, 0.1%TFA, gradient 10-100%) to give the title compound. $^1H$ NMR (300 MHz, $CDCl_3$) $\delta$ ppm: 8.39 (s, 1 H), 3.75 (d, 2H), 2.22 (m, 1 H), 0.96 (d, 6H). [ES+ MS] m/z 270 $(M)^+$.

**Intermediate 42: *N'*-(5-bromo-2-cyano-4-pyrimidinyl)-4-[(4-methyl-1-piperazinyl) methyl]-*N'*-(2-methylpropyl) benzohydrazide trifluoroacetate.**

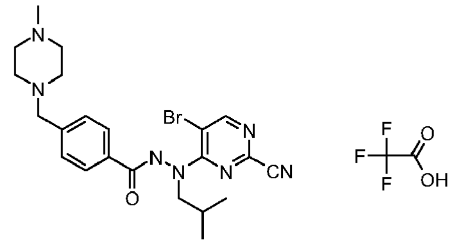

Preparation of 4-[(4-methyl-1-piperazinyl)methyl]benzoyl chloride.
Intermediate 38 (500 mg, 2.13 mmol) was dissolved in thionyl chloride (5 ml). The reaction mixture was refluxed for 6 hours. The solvent was evaporated *in* vacuo and the crude product was used without any further purification.

To a stirred solution of Intermediate 41 (200 mg, 0.74 mmol) in pyridine (10 mL), a mixture of the previously prepared acid chloride (539 mg, 2.13 mmol) and DIPEA (0.26 mL, 1.48 mmol) in dry THF (10 mL) was added and the resulting reaction mixture was stirred at room temperature for 2 hours. The solvent was evaporated *in* vacuo and the crude reaction mixture was purified by flash chromatography (silica gel, dichloromethane:methanol). The solid was repurified by HPLC ($H_2O$, 0.1 %TFA:ACN) to give the title compound. $^1H$ NMR (300 MHz, DMSO) δ ppm: 11.36 (s, 1 H), 8.64 (s, 1 H), 7.92 (d, 2H), 7.48 (d, 2H), 3.98 (m, 1 H), 3.73 (s 2H), 3.38 (m, 4H), 3.02 (m 4H), 2.78 (s, 3H) 2.42 (m, 1 H), 2.05 (m, 1 H), 0.94 (d, 6H). [ES+ MS] m/z 486 $(M)^+$.

**Examples**

**Example 1: *N'*-(2-cyano-9-methyl-9*H*-purin-6-yl)-*N'*-(cyclopentylmethyl)-4-{[4-(4-methy)-1-piperazinyl)-1-piperidinyl]methyl}benzohydrazide trifluoroacetate.**

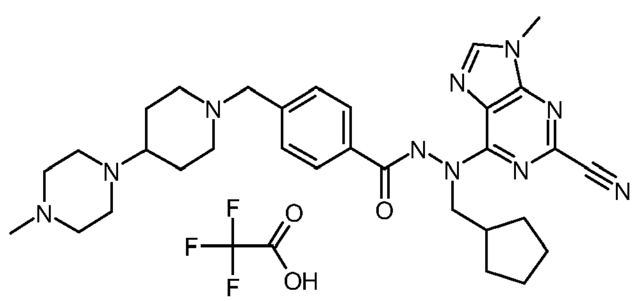

Intermediate 6 (125mg, 0.27mmol) was dissolved in ACN (10mL). Dry potassium carbonate (Aldrich, 45mg, 0.32mmol) and 1-methyl-4-(piperidin-4-yl)-piperazine (Fluorochem, 49mg, 0.27mmol) were added and the mixture was stirred at r.t. for about 5 h. The solution was then filtered and concentrated under reduced pressure. The crude product was purified by preparative HPLC (XTERRA 19x150 mm, ACN: H2O, 0.1%TFA, gradient 10-100%) to give the title compound. $^1H$ NMR (300 MHz, $CDCl_3$) δ ppm: 7.98 (d, 2H); 7.53 (d, 2H); 7.76-7.61 (br, 1 H); 4.51 (br, 1 H); 4.20 (s, 2H); 4.01 (br, 1 H); 3.78 (s, 3H); 3.50 (br, 2H); 3.36 (m, 1 H); 3.18 (br, 3H); 2.88 (br, 3H); 2.77 (s, 3H); 2.37 (m, 1 H); 2.00 -1.21 (m, 13H). [ES+MS] m/z 571 $(MH)^+$.

**Example 2: *N'*-(2-cyano-9-methyl-9*H*-purin-6-yl)-*N'*-(cyclopentylmethyl)-4-({4-[(1-methyl-4-piperidinyl)methyl]-1-piperazinyl}methyl)benzohydrazide trifluoroacetate.**

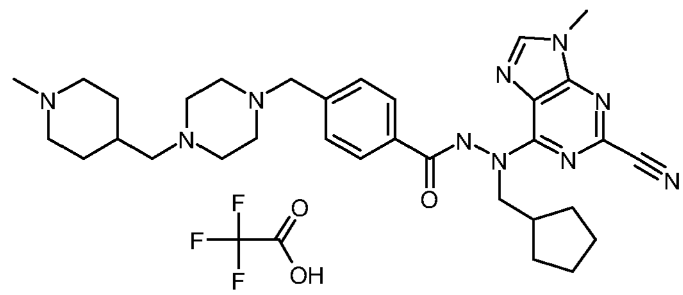

Intermediate 6 (50mg, 0.11mmol) was dissolved in ACN (10mL). Dry potassium carbonate (Aldrich, 18mg, 0.13mmol) and 1-(N-methyl-4-piperidin-methyl)-piperazine (Fluorochem, 22mg, 0.11mmol) were added and the mixture was stirred at r.t. for 1 h. The solution was then filtered and concentrated under reduced pressure. The residue product was purified by preparative HPLC (XTERRA 19x150 mm, ACN: H2O, 0.1 %TFA, gradient 10-100%) to give the title compound. $^1$H NMR (300 MHz, DMSO) δ ppm: 11.43-11.14 (m, 1H); 9.52 (br., 1 H); 8.51-8.25 (m, 1 H); 7.97 (m, 2H); 7.56 (m,2H); 4.75 -2.60 (m, 14H); 3.80 (s, 2H); 3.74 2 (s, 3H); 2.75 (s, 3H); 2.74 (s, 2H); 2.23 (m, 1 H); 1.96-121 (m, 13H). [ES+MS] m/z 585 $(MH)^+$.

**Example 3: *N'*-(2-cyano-9-methyl-9*H*-purin-6-yl)-*N'*-(cyclopentylmethyl)-4-({4-[(1-methyl-3-piperidinyl)methyl]-1-piperazinyl}methyl)benzohydrazide trifluoroacetate.**

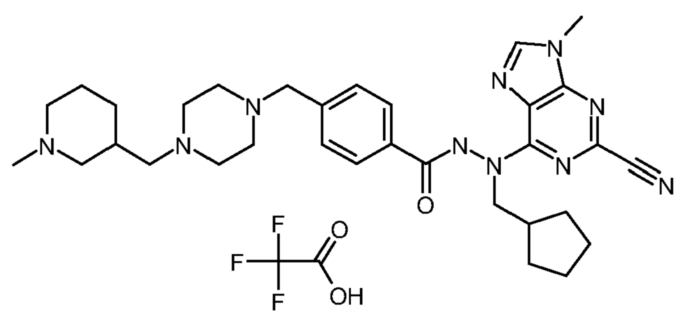

Intermediate 6 (50mg, 0.11mmol) was dissolved in ACN (10mL). Dry potassium carbonate (Aldrich, 18mg, 0.13mmol) and 1-(N-methyl-3-piperidyl-methyl)-piperazine (Fluorochem, 21 mg, 0.11mmol) were added and the mixture was stirred at r.t. for 1 h. The reaction crude was then filtered and concentrated under reduced pressure. The residue was purified by preparative HPLC (XTERRA 19x150 mm, ACN: H2O, 0.1%TFA, gradient 10-100%) to give the title compound. $^1$H NMR (300 MHz, DMSO) δ ppm: 11.45-11.17 (m, 1 H); 9.65 (br., 1 H); 8.51-8.25 (m, 1 H); 8.0 (m, 2H); 7.60 (m,2H); 4.75 -2.56 (m, 14H); 3.81 (s, 2H); 3.75 (s, 3H); 2.75 (s, 3H); 2.74 (s, 2H); 2.23 (m, 1 H); 1.96-121 (m, 13H). [ES+MS] m/z 585 $(MH)^+$.

**Example 4: *N'*-(2-cyano-9-methyl-9*H*-purin-6-yl)-*N'*-cyclopentyl-4-{[4-(4-methyl-1-piperazinyl)-1-piperidinyl] methyl}benzohydrazide trifluoroacetate.**

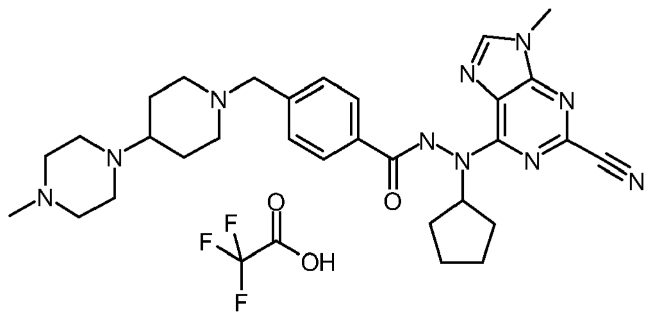

To a solution of intermediate 17 (165mg, 0.36mmol) in ACN (4mL), DIPEA (Fluka, 92μl, 0.54mmol) and 1-methyl-4-(piperidin-4-yl)-piperazine (Fluorochem, 80mg, 0.44mmol) were added. The resulting reaction mixture was stirred at r.t. and under $N_2$ atmosphere overnight. The solvent was evaporated under reduced pressure and the resulting crude was purified by preparative HPLC (SUNFIRE 30x150 mm, ACN:$H_2O$, 0.1%TFA, gradient 20-100%) to give the title compound. $^1$H NMR (300 MHz, DMSO-d6, 80°C) δ ppm: 10.86 (br.s, 1 H); 8.27 (br.s, 1 H); 8.02 (dd, 2H); 7.66 (dd, 2H); 5.54 (m, 1 H); 4.36 (s, 2H); 3.78 (s, 3H); 2.56-3.73 (m, 13H); 2.78 (s, 3H); 1.54-2.04 (m, 12H). [ES+MS] m/z 557 $(MH)^+$.

**Example 5: *N'*-(2-cyano-9-methyl-9*H*-purin-6-yl)-*N'*-cyclopentyl-4-({4-[(1-methyl-4-piperidinyl)methyl]-1-piperazinyl}methyl)benzohydrazide trifluoroacetate.**

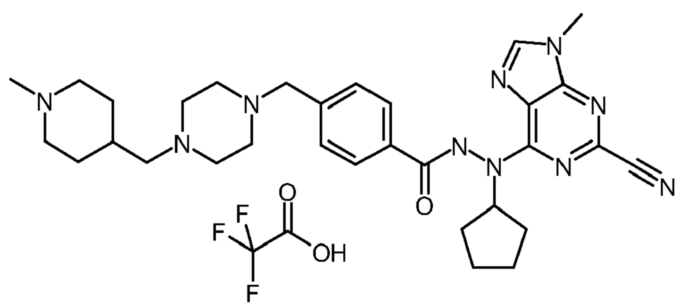

To a solution of intermediate 17 (143mg, 0.32mmol) in ACN (4mL), DIPEA (Fluka, 79μl, 0.47mmol) and 1-(N-methyl-4-piperidin-methyl)-piperazine (Fluorochem, 75mg, 0.38mmol) were added. The resulting reaction mixture was stirred at r.t. and under $N_2$ atmosphere overnight. The solvent was evaporated under reduced pressure and the resulting crude product was purified by preparative HPLC (SUNFIRE 30x150 mm, ACN:$H_2O$, 0.1%TFA, gradient 20-100%) to give the title compound. $^1$H NMR (300 MHz, DMSO-d6, 80°C) δ ppm: 10.80 (br.s, 1 H); 8.26 (br.s, 1 H); 7.96 (dd, 2H); 7.55 (dd, 2H); 5.58 (m, 1 H); 3.67-4.16 (m, 14H); 3.78 (s, 3H); 3.44 (m, 3H); 2.77 (s, 3H); 1.26-2.02 (m, 12H). [ES+MS] m/z 571 $(MH)^+$.

**Example 6: *N'*-(2-cyano-9-methyl-9*H*-purin-6-yl)-*N'*-cyclopentyl-4-({4-[(4-methyl-1-piperazinyl)carbonyl]-1-piperidinyl}methyl)benzohydrazide trifluoroacetate.**

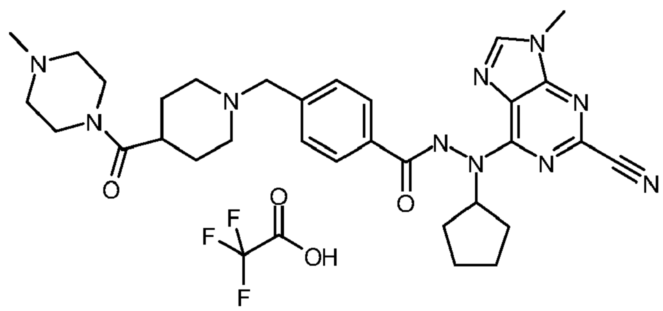

To a solution of intermediate 17 (135mg, 0.30mmol) in ACN (4mL), DIPEA (Fluka, 76μl, 0.45mmol) and (4-methyl-piperazin-1-yl)-piperidin-4-yl-methanone.2HCl (Fluorochem, 75mg, 0.36mmol) were added. The resulting reaction mixture was stirred at r.t. and under $N_2$ atmosphere overnight. The solvent was evaporated under reduced pressure and the resulting crude was purified by preparative HPLC (SUNFIRE 30x150 mm, ACN:$H_2O$, 0.1%TFA, gradient 20-100%) to give the title compound. $^1H$ NMR (300 MHz, DMSO-d6, 80˚C) δ ppm: 10.86 (br.s, 1 H); 8.27 (br.s, 1 H); 8.02 (dd, 2H); 7.67 (dd, 2H); 5.52 (m, 1 H); 4.35 (s, 2H); 3.78 (s, 3H); 2.54-3.81 (m, 13H); 2.78 (s, 3H); 1.54-2.03 (m, 12H). [ES+MS] m/z 585 $(MH)^+$.

**Example 7: *N'*-(2-cyano-9-methyl-9*H*-purin-6-yl)-*N'*-cyclopentyl-4-{[3-(1-pyrrolidinyl)-1-azetidinyl]methyl}benzohydrazide trifluoroacetate.**

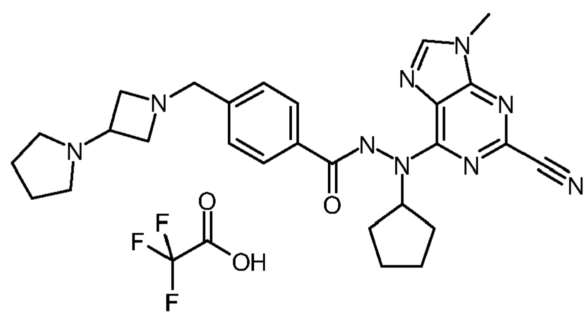

To a solution of intermediate 17 (132mg, 0.29mmol) in ACN (3mL), DIPEA (Fluka, 147μl, 0.87mmol) and intermediate 20 (69mg, 0.35mmol) were added. The resulting reaction mixture was stirred at r.t. and under $N_2$ atmosphere overnight. The solvent was evaporated under reduced pressure and the resulting crude was purified by preparative HPLC (SUNFIRE 30x150 mm, ACN:$H_2O$, 0.1%TFA, gradient 10-100%) to give the title compound. $^1H$ NMR (300 MHz, DMSO-d6, 80˚C) δ ppm: 10.79 (br.s, 1H); 8.26 (br.s, 1 H); 7.95 (dd, 2H); 7.51 (dd, 2H); 4.67 (m, 1 H); 4.10 (s, 2H); 3.51-4.02 (m, 14H); 3.08 (m, 2H); 1.54-2.03 (m, 8H). [ES+MS] m/z 500 $(MH)^+$.

**Example 8: *N' N'*-(2-cyano-9-methyl-9*H*-purin-6-yl)-*N'*-cyclohexyl-4-{[4-(4-methyl-1-piperazinyl)-1-piperidinyl] methyl}benzohydrazide trifluoroacetate.**

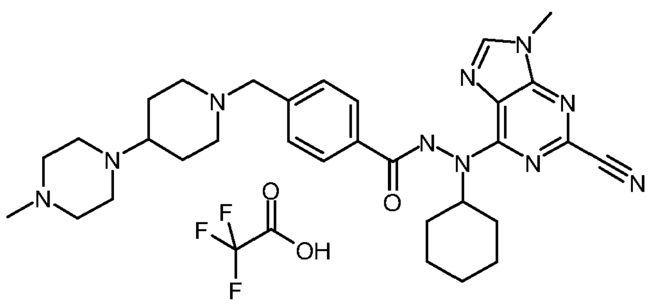

To a solution of intermediate 25 (161mg, 0.34mmol) in ACN (4mL), DIPEA (Fluka, 87µl, 0.51mmol) and 1-methyl-4-(piperidin-4-yl)-piperazine (Fluorochem, 75mg, 0.41mmol) were added. The resulting reaction mixture was stirred at r.t. and under $N_2$ atmosphere overnight. The solvent was evaporated under reduced pressure and the resulting crude was purified by preparative HPLC (SUNFIRE 30x150 mm, ACN:$H_2O$, 0.1%TFA, gradient 20-100%) to give the title compound. $^1H$ NMR (300 MHz, DMSO-d6, 80°C) δ ppm: 10.77 (br.s, 1 H); 8.26 (br.s, 1 H); 8.02 (dd, 2H); 7.65 (dd, 2H); 5.07 (m, 1 H); 4.31 (s, 2H); 3.78 (s, 3H); 2.83-3.74 (m, 13H); 2.76 (s, 3H); 1.03-2.02 (m, 14H). [ES+MS] m/z 571 $(MH)^+$.

**Example 9: *N'*-(2-cyano-9-methyl-9*H*-purin-6-yl)-*N'*-cyclohexyl-4-({4-[(1-methyl-4-piperidinyl)methyl]-1-piperazinyl}methyl)-benzohydrazide trifluoroacetate.**

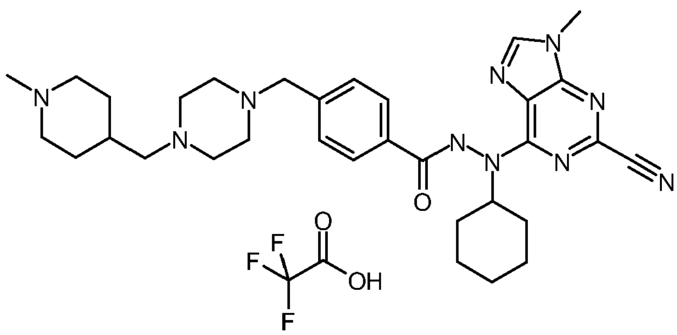

To a solution of Intermediate 25 (146mg, 0.31mmol) in ACN (4mL), DIPEA (Fluka, 79µl, 0.47mmol) and 1-(N-methyl-4-piperidin-methyl)-piperazine (Fluorochem, 73mg, 0.37mmol) were added. The resulting reaction mixture was stirred at r.t. and under $N_2$ atmosphere overnight. The solvent was evaporated under reduced pressure and the resulting crude was purified by preparative HPLC (SUNFIRE 30x150 mm, ACN:$H_2O$, 0.1%TFA, gradient 20-100%) to give the title compound. $^1H$ NMR (300 MHz, DMSO-d6, 80°C) δ ppm: 10.72 (br.s, 1 H); 8.27 (br.s, 1 H); 7.98 (dd, 2H); 7.55 (dd, 2H); 4.90 (m, 1 H); 4.01 (br.s, 2H); 3.78 (s, 3H); 2.81-3.73 (m, 15H); 2.77 (s, 3H); 0.99-2.03 (m, 14H). [ES+MS] m/z 585 $(MH)^+$.

**Example 10: *N'*-(2-cyano-9-methyl-9*H*-purin-6-yl)-*N'*-cyclohexyl-4-({4-[(4-methyl-1-piperazinyl)carbonyl]-1-piperidinyl}methyl)benzohydrazide trifluoroacetate.**

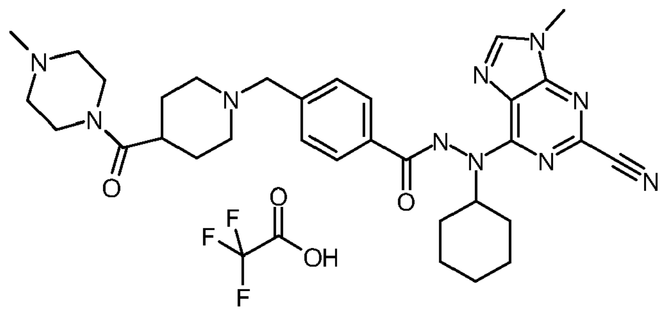

To a solution of Intermediate 25 (149mg, 0.32mmol) in ACN (4mL), DIPEA (Fluka, 81μl, 0.48mmol) and (4-methyl-piperazin-1-yl)-piperidin-4-yl-methanone.2HCl (Fluorochem, 81 mg, 0.38mmol) were added. The resulting reaction mixture was stirred at r.t. under $N_2$ atmosphere overnight. The solvent was evaporated under reduced pressure and the resulting crude was purified by preparative HPLC (SUNFIRE 30x150 mm, ACN:$H_2O$, 0.1%TFA, gradient 20-100%) to give the title compound. $^1H$ NMR (300 MHz, DMSO-d6, 80˚C) δ ppm: 10.80 (br.s, 1 H); 8.26 (br.s, 1 H); 8.03 (dd, 2H); 7.66 (dd, 2H); 5.01 (m, 1 H); 4.37 (s, 2H); 3.78 (s, 3H); 2.86-3.60 (m, 13H); 2.79 (s, 3H); 0.99-2.03 (m, 14H). [ES+MS] m/z 599 $(MH)^+$.

**Example 11: *N'*-(2-cyano-9-methyl-9*H*-purin-6-yl)-*N'*-cyclohexyl-4-{[3-(1-pyrrolidinyl)-1-azetidinyl]methyl}benzohydrazide triflouroacetate.**

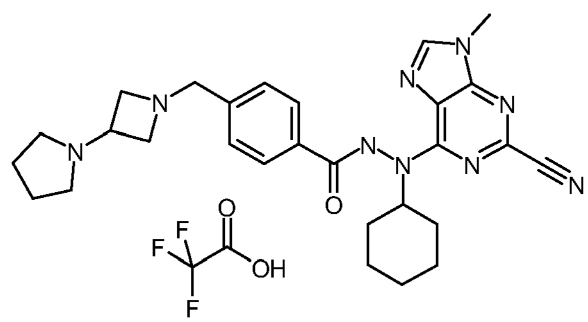

To a solution of Intermediate 25 (156mg, 0.33mmol) in ACN (3mL), DIPEA (Fluka, 168μl, 0.99mmol) and Intermediate 20 (80mg, 0.4mmol) were added. The resulting reaction mixture was stirred at r.t. under $N_2$ atmosphere overnight. The solvent was evaporated under reduced pressure and the resulting crude product was purified by preparative HPLC (SUNFIRE 30x150 mm, ACN:$H_2O$, 0.1%TFA, gradient 10-100%) to give the title compound. $^1H$ NMR (300 MHz, DMSO-d6, 80˚C) δ ppm: 10.72 (br.s, 1 H); 8.25 (br.s, 1 H); 7.96 (dd, 2H); 7.52 (dd, 2H); 4.67 (m, 1 H); 4.13 (s, 2H); 3.65-4.06 (m, 12H); 3.10 (m, 4H); 1.54-2.03 (m, 10H). [ES+MS] m/z 514 $(MH)^+$.

**Example 12: 1,1-dimethylethyl 2-{2-cyano-9-[3-(dimethylamino)propyl]-9*H*-purin-6-yl}-2-(cyclopentylmethyl) hydrazinecarboxylate trifluoroacetate.**

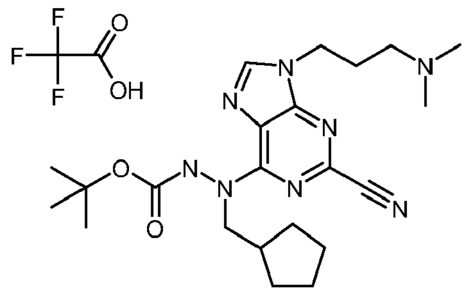

The preparation of this compound has been described as Intermediate 26.

**Example 13: 1,1-dimethylethyl 2-{2-cyano-9-[2-(dimethylamino)ethyl]-9*H*-purin-6-yl}-2-(cyclopentylmethyl)hydrazinecarboxylate trifluoroacetate.**

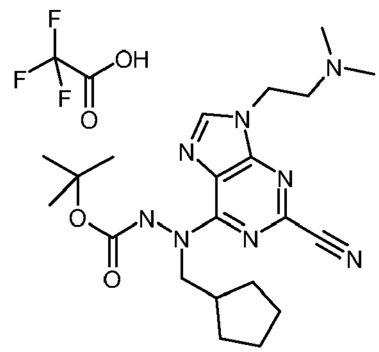

The preparation of this compound has been described as Intermediate 27.

**Example 14: *N*'-{2-cyano-9-[2-(dimethylamino)ethyl]-9*H*-purin-6-yl}-*N*'-(cyclo pentylmethyl)-4-[(4-methyl-1-piperazinyl)methyl]benzohydrazide trifluoroacetate.**

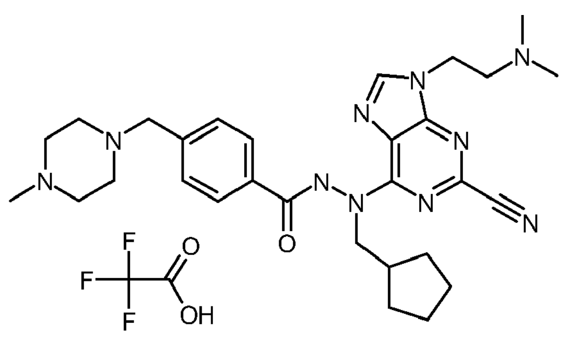

Intermediate 28 (crude, 0.7mmol) was dissolved in THF (30mL). DIPEA (Fluka, 0.24mL, 1.4mmol) and 4-bromomethyl benzoyl bromide (Aldrich, 185mg, 0.67mmol) were added. The mixture was stirred at r.t. for 1 h. N-Methylpiperazine (Aldrich, 0.15mL., 1.4mmol) and DIPEA (Fluka, 0.24mL, 1.4mmol) were added and the mixture was stirred at r.t.. The solvent was removed and the crude product was purified by preparative HPLC (XTERRA 19x150 mm,

ACN: H2O, 0.1%TFA, gradient 10-100%) to give the title compound. $^1$H NMR (300 MHz, DMSO 80˚C) δ ppm: 11.06 (br, 1 H); 8.37 (br, 1 H); 7.94 (d, 2H); 7.48 (d, 2H); 4.60 (m, 2H); 4.0 -2.56 (m, 14H); 3.70 (s, 3H); 2.86 (s, 6H); 2.79 (s, 4H); 1.85 (m, 1 H); 1.65 -1.32 (m, 8H). [ES+MS] m/z 545 (MH)$^+$.

**Example 15: 1,1-dimethylethyl 2-{2-cyano-9-[3-(dimethylamino)propyl]-9*H*-purin-6-yl}-2-cyclopentylhydrazinecarboxylate trifluoroacetate.**

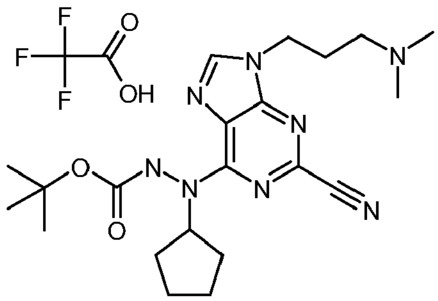

Intermediate 14 (200mg, 0.5mmol) was dissolved in anhydrous DMF (10mL) under argon and anhydrous potassium carbonate (138mg. 1mmol) was added. The mixture was stirred at r.t. and 3-dimethylaminopropyl chloride hydrochloride (Aldrich, 87mg. 0.55mmol) were added. The mixture was stirred at r.t for 2 days. The mixture was filtered and the solvent removed under reduced pressure. The crude was purified by preparative HPLC (SUNFIRE 19x150 mm, ACN: H2O, 0.1 %TFA, gradient 10-100%) to give the title compound. $^1$H NMR (300 MHz, DMSO 80˚C) δ ppm: 9.29 (br. 1 H); 8.42 (s, 1 H); 5.68 (br. 1 H); 4.30 (t, 2H); 3.12 (m, 2H); 2.78 (s, 6H); 2.23 (m, 2H); 1.86-1.25 (m, 17H). [ES+MS] m/z 429 (MH)$^+$.

**Example 16: 1,1-dimethylethyl 2-{2-cyano-9-[3-(dimethylamino)propyl]-9*H*-purin-6-yl}-2-(cyclopentylmethyl)-1-[3-(dimethylamino)propyl]hydrazinecarboxylate trifluoroacetate.**

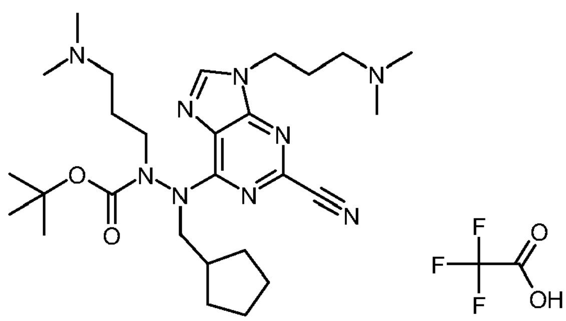

Intermediate 10 (50mg., 0.14mmol) was dissolved in dry DMF (5mL) under nitrogen. Anhydrous potassium carbonate (39mg., 0.28mmol) and 3-di-methyl-amino-propylchloride.HCl (Aldrich, 30mg, 0.21 mmol) were added and the mixture was stirred at 50˚C overnight. The solution was filtered and concentrated under reduced pressure. The crude was dissolved in DCM, washed with sat $NH_4Cl$ and brine. The organic layer was dried over anhydrous sodium sulphate. The crude was purified by preparative HPLC (SUNFIRE 19x150 mm, ACN: H2O, 0.1%TFA, gradient 10-100%) to give the title compound. $^1$H NMR (300 MHz, DMSO 80˚C) δ ppm: 9.57 (br, 1 H); 8.50 (s, 1 H); 4.31 (t, 2H); 3.72 (br. 1 H); 3.16 (m, 4H); 2.78 (s, 12H); 2.38 (m, 1 H); 2.25 (m, 2H); 2.02 (m, 2H); 1.83-1.10 (m, 17H). [ES+MS] m/z 528 (MH)$^+$.

**Example 17: *N'*-{2-cyano-9-[3-(dimethylamino)propyl]-9*H*-purin-6-yl}-*N'*-cyclo pentyl-4-[(4-methyl-1-piperazinyl)methyl]benzohydrazide trifluoroacetate.**

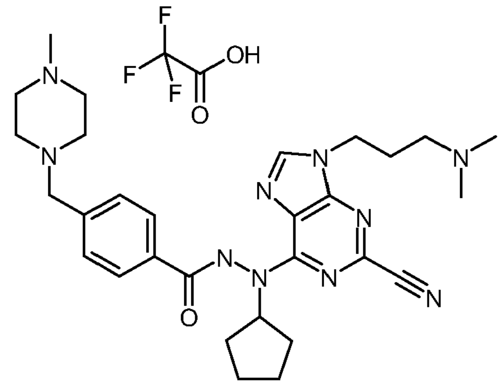

Intermediate 29 (28mg., 0.08mmol) was dissolved in anhydrous THF (10mL) under $N_2$. 4-Bromomethyl benzoyl bromide (Aldrich, 21 mg, 0.07mmol) and DIPEA (Fluka, 27.4mL, 0.16mmol) were added. The mixture was stirred at r.t. for 45 min. Then, N-methylpiperazine (Aldrich, 1.1 mL, 0.09mmol) in THF (5 mL) was added and the mixture was stirred overnight. The solvent was removed under reduced pressure, dissolved in DCM and washed successively with sat. $NaHCO_3$ and brine. The organic layer was dried over anhydrous sodium sulphate and the solvent was evaporated under reduced pressure. The crude was purified by preparative HPLC (XTERRA 19x150 mm, ACN: H2O, 0.1%TFA, gradient 10-100%) to give the title compound. $^1$H NMR (300 MHz, DMSO 80˚C) δ ppm: 10.75 (br. 1 H); 9.36 (br. 1 H); 8.35 (s, 1 H); 7.92 (d, 2H); 7.48 (d, 2H); 5.65 (br, 1 H); 4.29 (t, 2H); 3.70 (s,2H); 2.81-2.78 (m, 11 H); 2.23 (m, 3H); 1.90 (m, 4H); 1.61 (m, 4H). [ES+MS] m/z 545 $(MH)^+$.

Similarly prepared:

**Example 18:** N'-(2-cyano-9-methyl-9H-purin-6-yl)-N'-(cydopentylmethyl)-4-{[4-(1-methyl-4-piperidinyl)-1-piperazinyl] methyl}benzohydrazide trifluoroacetate; [ES+MS] m/z 571 (MH)+

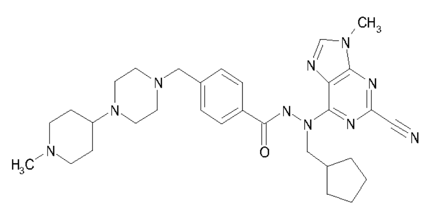

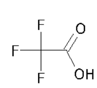

**Example 19:** 1,1-dimethylethyl 2-{2-cyano-9-[3-(dimethylamino)propyl]-9H-purin-6-yl}-2-(tetrahydro-2H-pyran-4-yl)hydrazinecarboxylate trifluoroacetate; [ES+MS] m/z 445 (MH)+

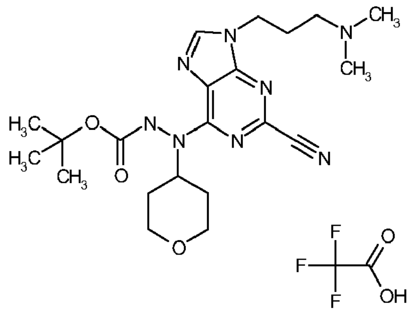

**Example 20**: 1,1-dimethylethyl-2-(1-acetyl-4-piperidinyl)-2-(2-cyano-9-methyl-9H-purin-6-yl)hydrazinecarboxylate; [ES+MS] m/z 415 (MH)+

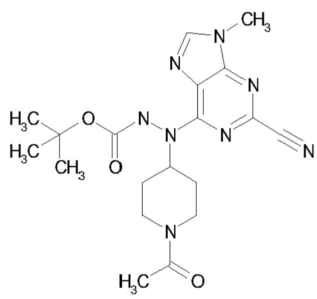

**Example 21:** 1,1-dimethylethyl-2-{2-cyano-9-[2-(1,3-dioxo-1,3-dihydro-2H-isoindol-2-yl)ethyl]-9H-purin-6-yl}-2-(cyclopentylmethyl)hydrazinecarboxylate; [ES+MS] m/z 531 (MH)+

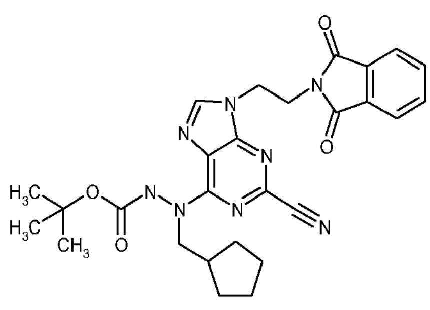

**Example 22:** N'-(2-cyano-9-methyl-9H-purin-6-yl)-4-[(4-methyl-1-piperazinyl)methyl]-N'-(2-methylpropyl)benzohydrazide; [ES+MS] m/z 462 (MH)+

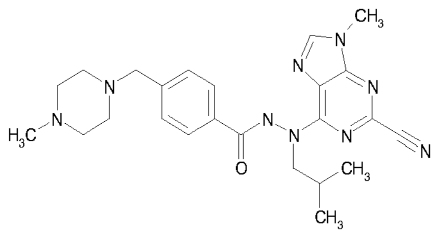

**Example 23:** N'-(2-cyano-9-methyl-9H-purin-6-yl)-N'-(cyclopentylmethyl)-4-[(4-methyl-1-piperazinyl)methyl]benzohydrazide trifluoroacetate; [ES+MS] m/z 488 (MH)+

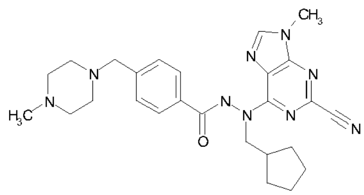

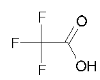

**Comparative Example 24: *N'*-(2-cyano-4-pyrimidinyl)-*N'*-(2,2-dimethylpropyl)-4-[(4-methyl-1-piperazinyl)methyl]benzohydrazide trifluoroacetate**

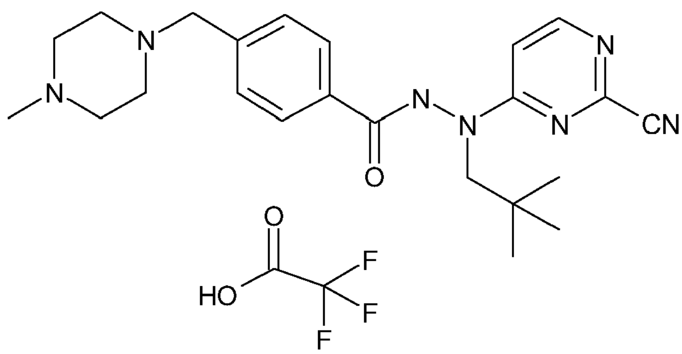

To a solution of Intermediate 38 (50mg, 0.20 mmol) in DCM (10 ml) was added DCC (ALDRICH, 163 mg, 0.8 mmol) and stirred at room temperature for 1 hour. To this solution was added Intermediate 37 (45 mg, 0.22 mmol) and stirring at room temperature overnight. Then DCC (ALDRICH, 41 mg, 0.20 mmol) and triethylamine (ALDRICH, 0.31μl, 0.22 mmol) were added and stirred at room 7 hours. After that, triethylamine (ALDRICH, 0.31 μl, 0.22 mmol) was added to give basic pH and the reaction was stirred at room temperature overnight. The reaction was evaporated and the product purified by column chromatography DCM/MeOH mixtures 100:0 to 0:100. The product was purified by preparative HPLC (Luna 21x250 mm, ACN:$H_2O$, 0.1% TFA, gradient 25-100%) to give the title compound. 1 H NMR (DMSO-d6, 300 MHz) δ ppm: 11.16 (m, 1 H), 9.45 (m, 1 H), 8.36 (m, 2H), 7.90 (d, 2H), 7.50 (d, 2H), 6.88 (br., 1 H), 4.09-4.16 (m, 1 H), 2.90-3.35 (br.m, 7H), 2.77 (s, 3H), 2.25-2.39 (m, 2H), 0.99 (s, 9H). [ES+ MS] m/z 422 (MH)+.

To a stirred solution of Intermediate 41 (200 mg, 0.70 mmol) in pyridine (1 mL) and DIPEA (5 mL), potassium carbonate (193 mg, 1.40 mmol) and previously obtained acid chloride (443 mg, 1.75 mmol) were added and the resulting reaction mixture was stirred at room temperature for 17 hours. The solvent was evaporated *in vacuo* and the crude

reaction mixture was purified by flash chromatography (silica gel, dichloromethane:methanol). The solid was repurified by HPLC ($H_2O$:ACN) to give the title compound. $^1H$ NMR (300 MHz, DMSO) δ ppm: 11.33 (s, 1 H), 8.64 (s, 1 H), 7.91 (d, 2H), 7.49 (d, 2H), 3.72 (s, 2H), 3.37 (m, 2H), 3.25-2.81 (br, 6H), 2.78 (s, 3H) 0.99 (s, 9H). [ES+ MS] m/z 500 ($MH^+$).

**Comparative Example 25: N'-(5-bromo-2-cyano-4-pyrimidinyl)-N'-isobutylbenzohydrazide**

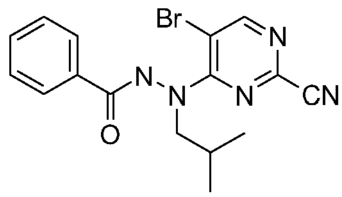

The title compound was prepared by a method analogous to that described for Intermediate 42, replacing the acid chloide of Intermediate 38 with benzoyl chloride. $^1H$ NMR (300 MHz, $CDCl_3$) δ ppm: 8.47 (s, 1 H); 8.18 (s, 1 H); 7.85-7.80 (m, 2H); 7.67-7.42 (m, 3H); 3.90-3-70 (m, 2H); 2.20-2.00 (m, 1 H); 1.03 (d, J=6.6 Hz, 6H). [ES+ MS] m/z 374 $(M)^+$.

**Biological Assays**

The compounds of this invention may be tested in one of several biological assays to determine the concentration of compound which is required to have a given pharmacological effect.

1) Determination of Falcipain-2, Falcipain-3 and Vivapain-2, proteolytic catalytic activity

Assays for Falcipain-2, Falcipain-3, and Vivapain-2 are carried out with parasitic recombinant enzymes. Standard assay conditions for the determination of kinetic constants used a fluorogenic peptide substrate, typically H-D-VLR-AFC (Falcipain-2, Falcipain-3, Vivapain-2 and are determined in 100 mM sodium acetate, pH 5.5, containing 10 mM DTT and 0.5 mM CHAPS (Falcipain-2, Falcipain-3, Vivapain-2), and 100 mM sodium acetate, pH 5.5, containing 5 mM L-cysteine, 1mM CHAPS and 50mM MES, pH 6.5, containing 0.5mM CHAPS, 10mM L-CYS. Stock substrate solutions are prepared at 20 mM in DMSO. The activity assays contained 30 uM substrate (Falcipain-2, Falcipain-3, Vivapain-2). All assays contained 1% DMSO. Independent experiments found that this level of DMSO had no effect on enzyme activity or kinetic constants. All assays are conducted at ambient temperature as end point assays being quenched after 60 minutes at 90 minutes, with 16.6 uM E-64 in 1 % DMSO. Product formation (AFC or AMC) is determined from fluorescence (excitation at 405nM; emission at 530nM, AFC, or excitation at 360 nM; emission at 460 nM, AMC) monitored with a LJL Aquest (Molecular Devices) fluorescent plate reader. In the case of kinetic reads (used in mechanism of action studies), the reaction is not quenched but is read in the plate reader every 3 minutes for approximately 90 minutes. In addition, the mechanism of action studies for Falcipain-2 utilize Z-LR-AMC as the substrate. Product formation is determined from the fluorescence of AMC, measured with a LJL Acquest (Molecular Devices) fluorescent plate reader (excitation at 360nM; emission at 460nM).

Inhibition studies

Potential inhibitors are evaluated using the quenched read (endpoint) method. Assays are carried out in the presence of variable concentrations of test compound. Reactions are initiated by addition of enzyme and substrate to wells containing inhibitor stamped in 100% DMSO. For endpoint assays, the reaction is quenched with the addition of E64. Dose response data is fit to an $IC_{50}$ curve with preset fitting tools according to equation 1:

$$y = a + (b-a)/(1+(10^x/10^c)^d) \qquad (1)$$

where y is the response at a particular inhibitor concentration x, a is the minimum response value, b is the maximum response value, c is the $IC_{50}$, and d is the slope of the $IC_{50}$ curve. Assuming the compound is a competitive inhibitor, the apparent $K_I$ can be calculated from $IC_{50}$, as shown in equation 2:

$$IC_{50} = appK_I\ (1+[S]/\ K_M) \qquad (2)$$

where $appK_I$ is the apparent $K_I$, S is the concentration of substrate, $K_M$ is the Michaelis binding constant for substrate, and $K_I$ is the binding constant of a competitive inhibitor for free enzyme. For a more direct measurement of the $K_I$ and the binding mechanism, we performed mechanism of action studies that included a titration of substrate and inhibitor with a kinetic read. If the progress curves for each of these kinetic assays are linear, the measured rates (v) were fit to equation 3:

$$v = V_m S\ /\ [(K_M\ (1 + [I]\ /\ K_I) + [S]\ (\ 1+ [I]\ /\ \alpha K_I)] \qquad (3)$$

where $V_m$ is the maximum velocity, S is the concentration of substrate with Michaelis constant of $K_M$, $[I]$ is the concentration of inhibitor, $K_I$ is the binding constant of inhibitor for free enzyme, and $\alpha K_I$ is the binding constant of inhibitor for a potential enzyme-substrate complex.

For those compounds whose progress curves were nonlinear, with a decrease in enzyme activity over time characteristic of time-dependent inhibition, the progress curves were fit to equation 4 to yield the $k_{obs}$:

$$[AMC] = v_S\ t + (v_0 - v_{SS})\ [1 - exp\ (-k_{obs}t)]\ /\ k_{obs} \qquad (4)$$

where [AMC] is the concentration of product formed over time $t$, $v_0$ is the initial reaction velocity and $v_S$ is the final steady state rate. The $k_{obs}$ values were fit to equations 5 and 6, describing a one-step and two-step time dependent binding mechansim respectively:

$$k_{obs} = k_{off}\ (\ 1 + [I]\ /\ appK_I) \qquad (5)$$

$$k_{obs} = k_{off} + k_{on}\ (\ [I]\ /\ (\ appK_I + [I]\ ) \qquad (6)$$

$$appK_I = K_I\ (1+[S]/\ K_M) \qquad (7)$$

Equation 7 describes the apparent $K_I$ for competitive compounds and was substituted into equations 5 and 6 to generate the relevant binding constants from the fitting routine. In addition, the initial and final velocities were fit to equation 3 to further define the binding mechanism and potency. A complete discussion of this kinetic treatment has been fully described (Morrison et al., Adv. Enzymol. Relat. Areas Mol. Biol., 1988, 61, 201).

2) Determination of whole cell activity against the *Plasmodium falciparum* parasite

Compounds can be evaluated for whole cell actvity against the *Plasmodium falciparum* parasite according to the procedure described in Sijwali S. and Rosenthal P. J., (2004) Proceedings of the National Academy of Sciences of the United States of America (PNAS) 101 (13), 4384-4389 (see in particular "Measurement of Parasite Growth rates and Inhibitor Sensitivity" on page 4385); $IC_{50}$ values can be calculated as described in Singh A. and Rosenthal P. J., (2001) Antimicrobial Agents and Chemotherapy 45(3), 949-951 (see in particular page 950, first column); synchronised

parasites can be prepared as described in Divo A. A. et al., (1985) Protozool. 32, 59-64.

3) Comparator compounds

Three compounds were employed as comparator compounds. Comparative Example 24 which is a trifluoroacetate salt, and comparative Example 25, which is the free base, were prepared as described hereinabove.
The free bases of each of these compounds are disclosed in WO 2005/103012 A1 (page 122-123, Example 15; and page 130, Example 17(4) respectively).

**Comparative Example 24: *N*'-(2-cyano-4-pyrimidinyl)-*N*'-(2,2-dimethylpropyl)-4-[(4-methyl-1-piperazinyl)methyl]benzohydrazide trifluoroacetate**

**Comparative Example 25: N'-(5-bromo-2-cyano-4-pyrimidinyl)-N'-isobutylbenzohydrazide**

It will be understood by the skilled artisan that under the ezymatic assay conditions described hereinabove, the assay result obtained for the free base of a given compound is expected to be the same as that obtained when a salt of that compound is tested. This is because the buffer used in the assay determines the pH under which the compound is tested; the pH determines the relative amounts of free base to salt of the compound being tested. This has been confirmed by testing in the enzymatic assays the free base, the hydrochloride salt and the trifluoroacetate salt of certain compounds of the type exemplified herein.

Results of falcipain-2 and falcipain-3 enzymatic assays, and whole cell assay

Examples 1-16 and 18-23, and comparative Examples 24 and 25 were tested in the enzymatic assays for falcipain-2 according to the procedure described hereinabove.
Examples 1, 2, 4-16 and 18-23, and comparative Examples 24 and 25 were tested in the enzymatic assays for falcipain-3 according to the procedure described hereinabove.
All exemplified compounds (Examples 1-23 and comparative Examples 24 and 25) were tested in the whole cell assay according to the procedure described hereinabove.
The results of falcipain-2 and falcipain-3 enzymatic assays, and the whole cell assay for the exemplified compounds (Examples 1-23 and comparative Examples 24 and 25) are shown in the table below.

**Table of falcipain-2, falcipain-3 and whole cell assay activities**

| Structure | Example No. | Falcipain-2 | Falcipain-3 | Whole cell |
|---|---|---|---|---|
| 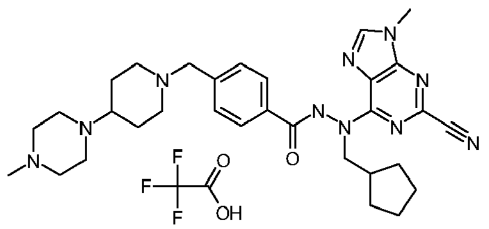 | 1 | C | D | D |
| 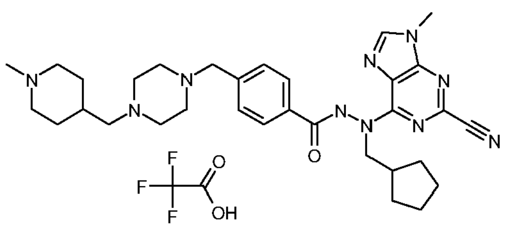 | 2 | C | D | D |

(continued)

| Structure | Example No. | Falcipain-2 | Falcipain-3 | Whole cell |
|---|---|---|---|---|
| 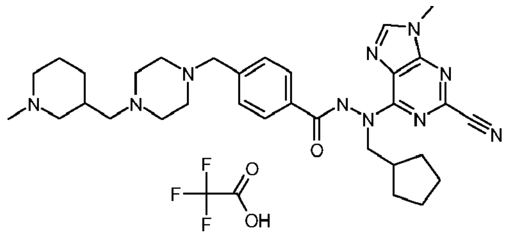 | 3 | C | D | D |
| 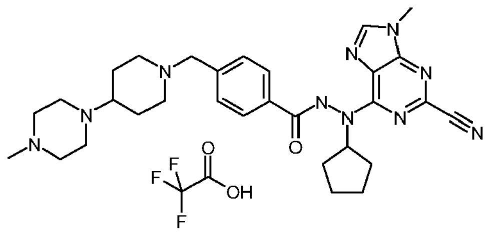 | 4 | C | E | C |
| 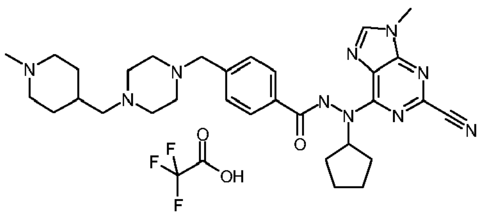 | 5 | C | F | C |
| 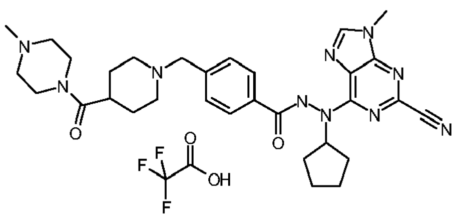 | 6 | C | E | D |
| 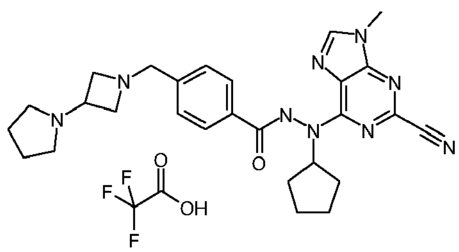 | 7 | C | F | D |
| 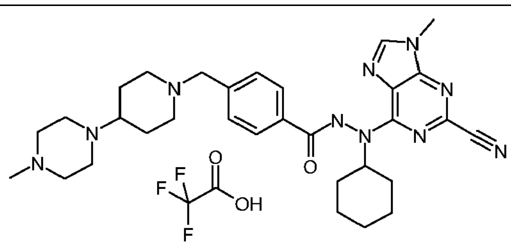 | 8 | B | D | C |

(continued)

| Structure | Example No. | Falcipain-2 | Falcipain-3 | Whole cell |
|---|---|---|---|---|
| 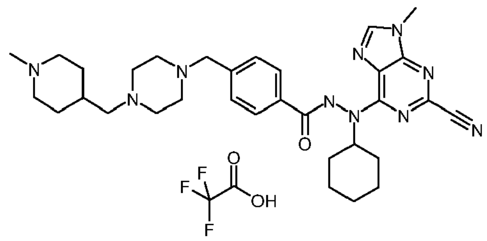 | 9 | C | D | C |
| 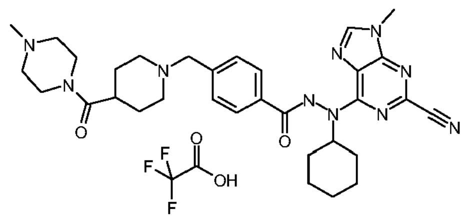 | 10 | C | D | C |
| 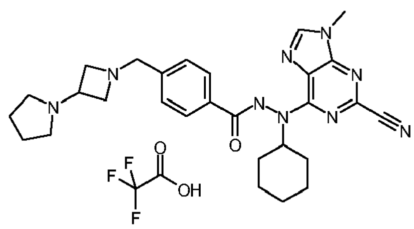 | 11 | C | D | D |
| 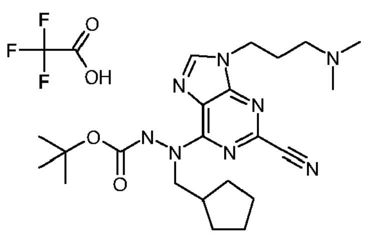 | 12 | B | D | D |
| 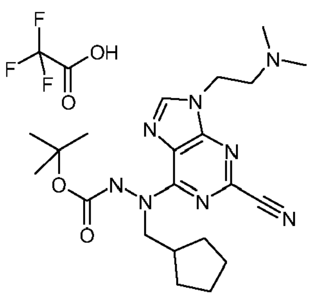 | 13 | A | D | D |
| 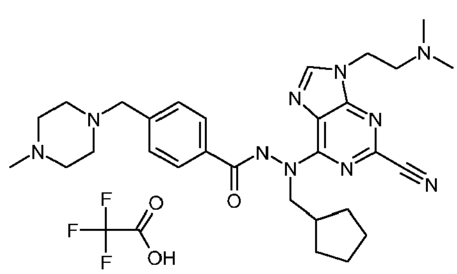 | 14 | C | D | D |

(continued)

| Structure | Example No. | Falcipain-2 | Falcipain-3 | Whole cell |
|---|---|---|---|---|
| 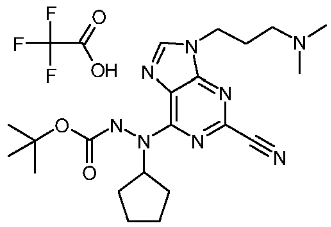 | 15 | C | D | D |
| 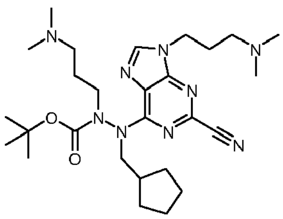 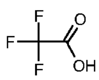 | 16 | C | E | C |
| 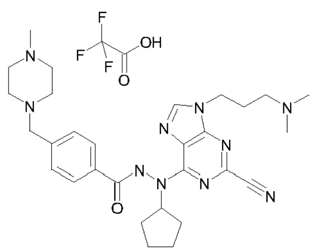 | 17 | - | - | D |
| 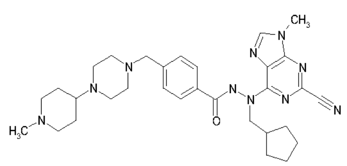 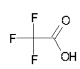 | 18 | C | D | D |
| 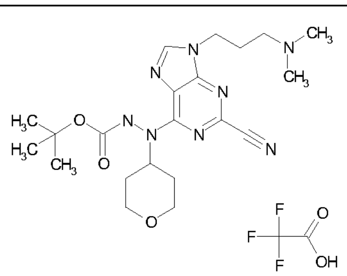 | 19 | C | D | D |
| 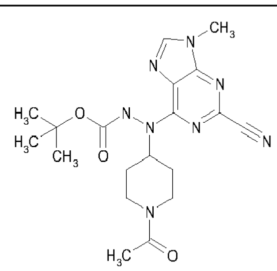 | 20 | B | E | E |

(continued)

| Structure | Example No. | Falcipain-2 | Falcipain-3 | Whole cell |
|---|---|---|---|---|
| 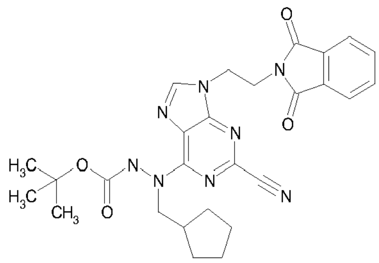 | 21 | D | F | F |
| 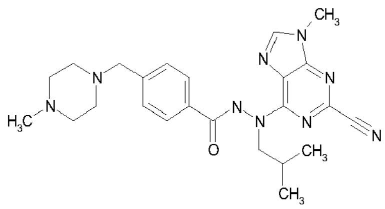 | 22 | D | F | G |
| 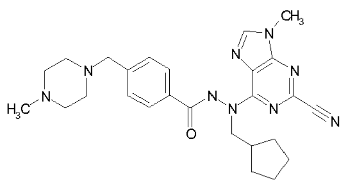 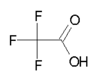 | 23 | C | D | G |
| 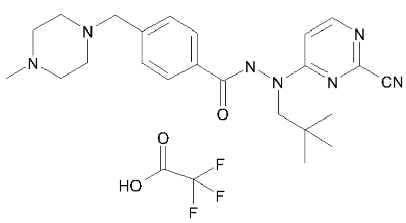 | Comparative 24 | D | F | H |
| 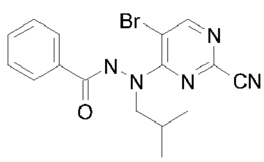 | Comparative 25 | A | D | H |

**Key to Table**

X = $IC_{50}$ in nM

X≤1 A
1 <X≤2.5 B
2.5<X≤15 C
15<X≤150 D
150<X≤250 E
250<X≤400 F
400<X≤1000 G
X>1000 H

The exemplified compounds of the invention exhibit an improved activity in the whole cell assay, as compared with comparative Examples 24 and 25 of the prior art.

## Claims

**1.** A compound of Formula I:

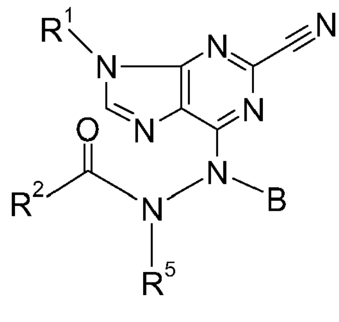

I

Wherein:

B represents

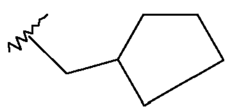

or

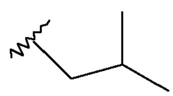

or

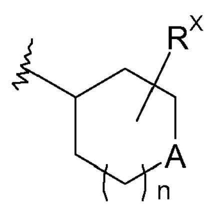

(i) (ii) (iii) ;

$R^1$ represents $C_{1-4}$alkyl, $C_{1-5}$alkylene-$NR^ER^F$ or $C_{1-2}$alkylene-N-phthalimide;
$R^5$ represents hydrogen or $C_{1-5}$alkylene-$NR^ER^F$;
$R^2$ represents -phenyl-$C_{1-3}$alkylene-X, -phenyl-$C_{1-3}$alkylene-X-$R^J$, -O$^t$Bu, pyridyl-phenyl-$C_{1-3}$alkylene-X, or pyridyl-phenyl-$C_{1-3}$alkylene-X-$R^J$,
wherein phenyl is optionally substituted with one group selected from halogen or $CF_3$; $R^J$ represents Z, -$C_{1-3}$alkylene-Z or -C(O)Z;
X and Z independently represent a monocyclic 4-, 5- or 6-membered, saturated hydrocarbon group containing one or two nitrogen atoms, which is optionally substituted with a group selected from: $C_{1-4}$alkyl, $C_{1-4}$alkylOH, OH and $NR^ER^F$;
Either A represents $CH_2$ and n represents 0 or 1; or A represents -O- or N(C(O)$C_{1-3}$alkyl) and n represents 1;
When A represents $CH_2$ and n represents 0, $R^x$ represents an optional methyl substituent on any carbon atom of the ring to which it is attached, otherwise $R^x$ is absent;
$R^E$ and $R^F$ independently for each occurrence represent hydrogen or $C_{1-4}$alkyl;
Provided that at least one of $R^1$, $R^2$, $R^5$ or B contains at least one nitrogen atom;
or a pharmaceutically acceptable derivative thereof.

**2.** A compound or a pharmaceutically acceptable derivative thereof according to claim 1 wherein B represents

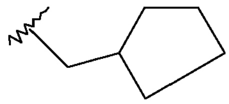 or 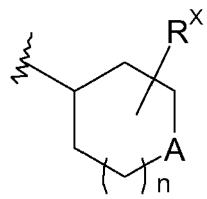

(i) (iii).

3. A compound or a pharmaceutically acceptable derivative thereof according to claim 1 or claim 2 wherein $R^1$ represents $C_{1-4}$alkyl or $C_{1-5}$alkylene-$NR^ER^F$.

4. A compound or a pharmaceutically acceptable derivative thereof according to any one of claims 1 to 3 wherein $R^2$ represents -phenyl-$C_{1-3}$alkylene-X, -phenyl-$C_{13}$alkylene-X-$R^J$ or -$O^tBu$.

5. A compound or a pharmaceutically acceptable derivative thereof according to any one of claims 1 to 4 wherein X represents an optionally substituted monocyclic 4- or 6-membered, saturated hydrocarbon group containing one or two nitrogen atoms..

6. A compound or a pharmaceutically acceptable derivative thereof according to any one of claims 1 to 5 wherein Z represents an optionally substituted monocyclic 5- or 6-membered, saturated hydrocarbon group containing one or two nitrogen atoms.

7. A compound or a pharmaceutically acceptable derivative thereof according to any one of claims 1 to 6 wherein A represents $CH_2$ and n represents 0 or 1.

8. A compound or a pharmaceutically acceptable derivative thereof according to any one of claims 1 to 7 wherein $R^E$ and $R^F$ independently for each occurrence represent $C_{1-4}$alkyl.

9. A compound selected from the list:

*N*'-(2-cyano-9-methyl-9*H*-purin-6-yl)-*N*'-(cyclopentylmethyl)-4-{[4-(4-methyl-1-piperazinyl)-1-piperidinyl]methyl}benzohydrazide;
*N*'-(2-cyano-9-methyl-9*H*-purin-6-yl)-*N*'-(cyclopentylmethyl)-4-({4-[(1-methyl-4-piperidinyl)methyl]-1-piperazinyl}methyl)benzohydrazide;
*N*'-(2-cyano-9-methyl-9*H*-purin-6-yl)-*N*'-(cyclopentylmethyl)-4-({4-[(1-methyl-3-piperidinyl)methyl]-1-piperazinyl}methyl)benzohydrazide;
*N*'-(2-cyano-9-methyl-9*H*-purin-6-yl)-*N*'-cyclopentyl-4-{[4-(4-methyl-1-piperazinyl)-1-piperidinyl]methyl}benzohydrazide;
*N*'-(2-cyano-9-methyl-9*H*-purin-6-yl)-*N*'-cyclopentyl-4-({4-[(1-methyl-4-piperidinyl)methyl]-1-piperazinyl}methyl)benzohydrazide;
*N*'-(2-cyano-9-methyl-9*H*-purin-6-yl)-*N*'-cyclopentyl-4-({4-[(4-methyl-1-piperazinyl)carbonyl]-1-piperidinyl}methyl)benzohydrazide;
*N*'-(2-cyano-9-methyl-9*H*-purin-6-yl)-*N*'-cyclopentyl-4-{[3-(1-pyrrolidinyl)-1-azetidinyl]methyl}benzohydrazide;
*N*'-(2-cyano-9-methyl-9*H*-purin-6-yl)-*N*'-cyclohexyl-4-{[4-(4-methyl-1-piperazinyl)-1-piperidinyl]methyl}benzohydrazide;
*N*'-(2-cyano-9-methyl-9*H*-purin-6-yl)-*N*'-cyclohexyl-4-({4-[(1-methyl-4-piperidinyl)methyl]-1-piperazinyl}methyl)-benzohydrazide;
*N*'-(2-cyano-9-methyl-9*H*-purin-6-yl)-*N*'-cyclohexyl-4-({4-[(4-methyl-1-piperazinyl)carbonyl]-1-piperidinyl}methyl)benzohydrazide;
*N*'-(2-cyano-9-methyl-9*H*-purin-6-yl)-*N*'-cyclohexyl-4-{[3-(1-pyrrolidinyl)-1-azetidinyl]methyl}benzohydrazide;
1,1-dimethylethyl 2-{2-cyano-9-[3-(dimethylamino)propyl]-9*H*-purin-6-yl}-2-(cyclopentylmethyl)hydrazinecarboxylate;
1,1-dimethylethyl 2-{2-cyano-9-[2-(dimethylamino)ethyl]-9*H*-purin-6-yl}-2-(cyclopentylmethyl)hydrazinecarboxylate;

*N*'-{2-cyano-9-[2-(dimethylamino)ethyl]-9*H*-purin-6-yl}-*N*'-(cyclo pentylmethyl)-4-[(4-methyl-1-piperazinyl)methyl]benzohydrazide;
1,1-dimethylethyl 2-{2-cyano-9-[3-(dimethylamino)propyl]-9*H*-purin-6-yl}-2-cyclopentylhydrazinecarboxylate;
1,1-dimethylethyl 2-{2-cyano-9-[3-(dimethylamino)propyl]-9*H*-purin-6-yl}-2-(cyclopentylmethyl)-1-[3-(dimethylamino)propyl]hydrazinecarboxylate;
*N*'-{2-cyano-9-[3-(dimethylamino)propyl]-9*H*-purin-6-yl}-*N*'-cyclopentyl-4-[(4-methyl-1-piperazinyl)methyl]benzohydrazide;
and pharmaceutically acceptable derivatives thereof.

**10.** A compound selected from the list:

*N*'-(2-cyano-9-methyl-9*H*-purin-6-yl)-*N*'-(cyclopentylmethyl)-4-{[4-(4-methyl-1-piperazinyl)-1-piperidinyl]methyl}benzohydrazide trifluoroacetate;
*N*'-(2-cyano-9-methyl-9H-purin-6-yl)-*N*'-(cyclopentylmethyl)-4-({4-[(1-methyl-4-piperidinyl)methyl]-1-piperazinyl}methyl)benzohydrazide trifluoroacetate;
*N*'-(2-cyano-9-methyl-9*H*-purin-6-yl)-*N*'-(cyclopentylmethyl)-4-({4-[(1-methyl-3-piperidinyl)methyl]-1-piperazinyl}methyl)benzohydrazide trifluoroacetate;
*N*'-(2-cyano-9-methyl-9*H*-purin-6-yl)-*N*'-cyclopentyl-4-{[4-(4-methyl-1-piperazinyl)-1-piperidinyl]methyl}benzohydrazide trifluoroacetate;
*N*'-(2-cyano-9-methyl-9*H*-purin-6-yl)-*N*'-cyclopentyl-4-({4-[(1-methyl-4-piperidinyl)methyl]-1-piperazinyl}methyl)benzohydrazide trifluoroacetate;
*N*'-(2-cyano-9-methyl-9*H*-purin-6-yl)-*N*'-cyclopentyl-4-({4-[(4-methyl-1-piperazinyl)carbonyl]-1-piperidinyl}methyl)benzohydrazide trifluoroacetate;
*N*'-(2-cyano-9-methyl-9*H*-purin-6-yl)-*N*'-cyclopentyl-4-{[3-(1-pyrrolidinyl)-1-azetidinyl]methyl}benzohydrazide trifluoroacetate;
*N*'-(2-cyano-9-methyl-9*H*-purin-6-yl)-*N*'-cyclohexyl-4-{[4-(4-methyl-1-piperazinyl)-1-piperidinyl]methyl}benzohydrazide trifluoroacetate;
*N*'-(2-cyano-9-methyl-9*H*-purin-6-yl)-*N*'-cyclohexyl-4-({4-[(1-methyl-4-piperidinyl)methyl]-1-piperazinyl}methyl)-benzohydrazide trifluoroacetate;
*N*'-(2-cyano-9-methyl-9*H*-purin-6-yl)-*N*'-cyclohexyl-4-({4-[(4-methyl-1-piperazinyl)carbonyl]-1-piperidinyl}methyl)benzohydrazide trifluoroacetate;
*N*'-(2-cyano-9-methyl-9H-purin-6-yl)-*N*'-cyclohexyl-4-{[3-(1-pyrrolidinyl)-1-azetidinyl]methyl}benzohydrazide trifluoroacetate;
1,1-dimethylethyl 2-{2-cyano-9-[3-(dimethylamino)propyl]-9*H*-purin-6-yl}-2-(cyclopentylmethyl)hydrazinecarboxylate trifluoroacetate;
1,1-dimethylethyl 2-{2-cyano-9-[2-(dimethylamino)ethyl]-9*H*-purin-6-yl}-2-(cyclopentylmethyl)hydrazinecarboxylate trifluoroacetate;
*N*'-{2-cyano-9-[2-(dimethylamino)ethyl]-9*H*-purin-6-yl}-*N*'-(cyclo pentylmethyl)-4-[(4-methyl-1-piperazinyl)methyl]benzohydrazide trifluoroacetate;
1,1-dimethylethyl 2-{2-cyano-9-[3-(dimethylamino)propyl]-9*H*-purin-6-yl}-2-cyclopentylhydrazinecarboxylate trifluoroacetate;
1,1-dimethylethyl 2-{2-cyano-9-[3-(dimethylamino)propyl]-9*H*-purin-6-yl}-2-(cyclopentylmethyl)-1-[3-(dimethylamino)propyl]hydrazinecarboxylate trifluoroacetate; and
*N*'-{2-cyano-9-[3-(dimethylamino)propyl]-9*H*-purin-6-yl}-*N*'-cyclopentyl-4-[(4-methyl-1-piperazinyl)methyl]benzohydrazide trifluoroacetate.

**11.** A compound or a pharmaceutically acceptable derivative thereof according to any one of claims 1 to 10 for use in medical therapy.

**12.** Use of a compound or a pharmaceutically acceptable derivative thereof according to any one of claims 1 to 10 in the manufacture of a medicament for the treatment of a condition susceptible to mediation by a cysteine protease inhibitor.

**13.** Use of a compound or a pharmaceutically acceptable derivative thereof according to any one of claims 1 to 10 in the manufacture of a medicament for the treatment of malaria.

**14.** A method for the treatment of a human or animal subject suffering from a condition susceptible to mediation by a cysteine protease inhibitor, comprising administering to said human or animal subject an effective amount of a

compound or a pharmaceutically acceptable derivative thereof according to any one of claims 1 to 10.

**15.** A method for the treatment of a human or animal subject suffering from malaria, comprising administering to said human or animal subject an effective amount of a compound or a pharmaceutically acceptable derivative thereof according to any one of claims 1 to 10.

**16.** A pharmaceutical composition comprising a compound or a pharmaceutically acceptable derivative thereof according to any one of claims 1 to 10 in admixture with one or more pharmaceutically acceptable carrier and/or excipient.

**17.** A process for the preparation of compounds of Formula I as defined in claim 1, wherein $R^5$ is hydrogen, from a reaction between compounds of Formula II, wherein $R^1$ and B are as defined for Formula I, compounds of Formula III, wherein Hal is chlorine, bromine or iodine, and compounds of Formula IV, wherein X and $R^J$ are as defined for Formula I, according to the Scheme below.

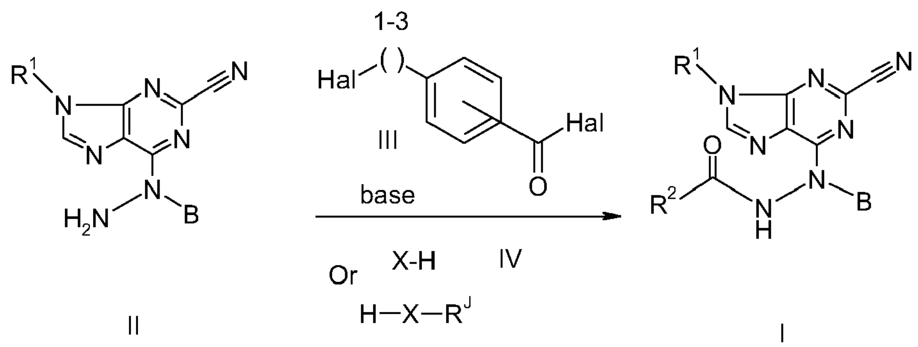


**18.** A process for the preparation of compounds of Formula I as defined in claim 1, wherein $R^5$ is hydrogen, from a reaction between compounds of Formula V, wherein $R^1$ and B are as defined for Formula I and Hal is chlorine, bromine or iodine, and compounds of Formula IV, wherein X and $R^J$ are as defined for Formula I, according to the Scheme below.

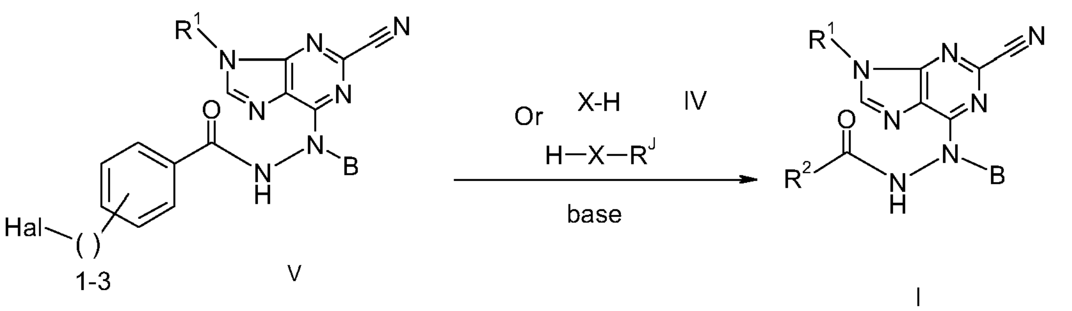


**19.** A process for the preparation of compounds of Formula I, wherein $R^5$ is hydrogen, from a reaction between compounds of Formula II and compounds of Formula VI, wherein X and $R^J$ are as defined for Formula I and Hal is chlorine, bromine or iodine, according to the Scheme below.

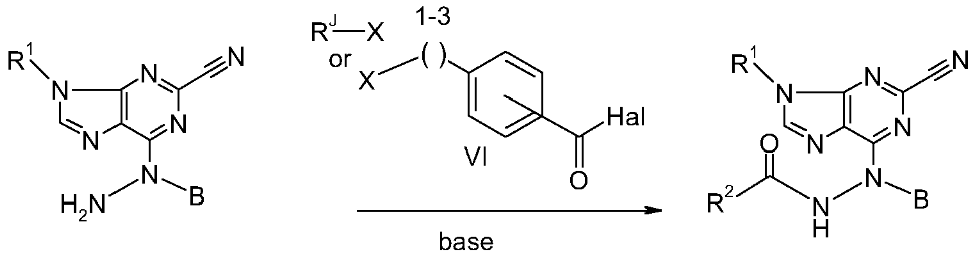


II

I

European Patent Office

## PARTIAL EUROPEAN SEARCH REPORT

which under Rule 45 of the European Patent Convention shall be considered, for the purposes of subsequent proceedings, as the European search report

Application Number
EP 07 38 1019

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | EP 1 739 081 A (ONO PHARMACEUTICAL CO [JP]) 3 January 2007 (2007-01-03)<br>* abstract *<br>* paragraph [0021] *<br>* sentence 20, paragraph 78 *<br>* examples *<br>* claims 1,8 *<br>----- | 1-19 | INV.<br>C07D473/34<br>A61K31/52<br>A61P33/06 |
| Y | WO 2006/027211 A (GLAXO GROUP LTD [GB]; CASTRO PICHEL JULIA [ES]; CHAPARRO MARTIN MARIA) 16 March 2006 (2006-03-16)<br>* abstract *<br>* page 1 *<br>* examples *<br>* claims *<br>----- | 1-19 | |
| A,D | EP 1 724 264 A (ONO PHARMACEUTICAL CO [JP]) 22 November 2006 (2006-11-22)<br>* abstract *<br>* paragraph [0019] *<br>* examples *<br>* claims *<br>-----<br>-/-- | 1-19 | TECHNICAL FIELDS SEARCHED (IPC)<br>C07D<br>A61K<br>A61P |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

2

| Place of search | Date of completion of the search | Examiner |
|---|---|---|
| Munich | 11 July 2007 | Stix-Malaun, Elke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C07)

European Patent Office

# PARTIAL EUROPEAN SEARCH REPORT

Application Number
EP 07 38 1019

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| A | WO 2004/000843 A (ASTRAZENECA AB [SE]; BAILEY ANDREW [GB]; PAIRAUDEAU GARRY [GB]; PATEL) 31 December 2003 (2003-12-31)<br>* abstract *<br>* examples *<br>* claims *<br>----- | 1-19 | |
| | | | TECHNICAL FIELDS SEARCHED (IPC) |

2

EPO FORM 1503 03.82 (P04C10)

European Patent Office

INCOMPLETE SEARCH SHEET C

Application Number
EP 07 38 1019

Although claims 14 and 15 are directed to a method of treatment of the human/animal body (Article 52(4) EPC), the search has been carried out and based on the alleged effects of the compound/composition.

-----

## ANNEX TO THE EUROPEAN SEARCH REPORT ON EUROPEAN PATENT APPLICATION NO. EP 07 38 1019

This annex lists the patent family members relating to the patent documents cited in the above-mentioned European search report.
The members are as contained in the European Patent Office EDP file on
The European Patent Office is in no way liable for these particulars which are merely given for the purpose of information.

11-07-2007

| Patent document cited in search report | | Publication date | Patent family member(s) | | Publication date |
|---|---|---|---|---|---|
| EP 1739081 | A | 03-01-2007 | WO | 2005103012 A1 | 03-11-2005 |
| WO 2006027211 | A | 16-03-2006 | AR | 050858 A1 | 29-11-2006 |
| | | | EP | 1632487 A1 | 08-03-2006 |
| EP 1724264 | A | 22-11-2006 | WO | 2005085210 A1 | 15-09-2005 |
| WO 2004000843 | A | 31-12-2003 | AU | 2003243096 A1 | 06-01-2004 |
| | | | EP | 1532148 A1 | 25-05-2005 |
| | | | JP | 2005533804 T | 10-11-2005 |
| | | | US | 2005203107 A1 | 15-09-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005085210 A1
- WO 2005103012 A1

### Non-patent literature cited in the description


- **BREMAN, J. G. et al.** *Am. Trop. Med. Hyg.,* 2001, vol. 64, 1-11
- **FRANCIS S.E. et al.** *Annu. Rev. Microbiol.,* 1997, vol. 51, 97-123
- Protease inhibitors. **ROSENTHAL P.J.** Antimalarial Chemotherapy: Mechanisms of Action, Resistance, and New Directions in Drug Discovery. Humana Press, 2001, 325-345
- **ROSENTHAL P. J. et al.** *J. Clin. Invest.,* 1998, vol. 82, 1560-6
- **GAMBOA DE DOMINGUEZ N.D. ; ROSENTHAL P.J.** *Blood,* 1996, vol. 87, 4448-54
- **ROSENTHAL P.J. ; NELSON R.G.** *Mol Biochem Parasitol,* 1992, vol. 51, 143-52
- **SALAS F. et al.** *Infect. Immun.,* 1995, vol. 63, 2120-5
- **ROSENTHAL, P. J. et al.** *Curr. Pharm. Des.,* 2002, vol. 8, 1659-1672
- **SHENAI B.R.** *J Biol Chem,* 2000, vol. 275, 29000-10
- **SHENAI B.R. et al.** *J. Biol. Chem.,* 2000, vol. 275, 29000-10
- **SIJWALI P.S. et al.** *Biochem. J.,* 2001, vol. 360, 481-9
- **SHENAI B.R. ; ROSENTHAL P.J.** *Mol. Biochem. Parasitol.,* 2002, vol. 122, 99-104
- **SIJWALI, P. S. et al.** *Proceedings of the National Academy of Sciences of the United States of America,* vol. 101, 8721-8726
- **MENDIS, K. ; SINA, B. J. ; MARCHESINI, P. ; CARTER, R.** The neglected burden of Plasmodium vivax malaria. *Am. J. Trop. Med. Hyg.,* 2001, vol. 64, 97-106
- **NA, B.K. ; SHENAI, B. R. ; SIJWALI, P. S. ; CHOE, Y. ; PANDEY, K. C. ; SINGH, A. ; CRAIK, C. S. ; ROSENTHAL, P. J.** identification and biochemical characterization of vivapains, cysteine proteases of the malaria parasite. *Plasmodium vivax. Biochem. J.,* 2004, vol. 378, 529-538
- Burger's Medicinal Chemistry and Drug Discovery. Principles and Practice. vol. 1
- **BERGE et al.** *J. Pharm. Sci.,* 1977, vol. 66, 1-19
- Remington's Pharmaceutical Sciences. Mack Publishing Company
- The Handbook of Pharmaceutical Additives. Gower Publishing Limited
- The Handbook of Pharmaceutical Excipients. the American Pharmaceutical Association and the Pharmaceutical Press
- **LUO G. et al.** *Tetrahedron Letters,* 2002, vol. 43 (33), 5739-5742
- **HILPERT, H.** *Tetrahedron,* 2001, vol. 57, 7675-7683
- **DYKER, H. et al.** *J. Org. Chem.,* 2001, vol. 66, 3760-3766
- **T.W. GREENE ; P.G.M. WUTS.** Protective groups in organic synthesis. John Wiley & sons, 1991
- **P.J. KOCIENSKI.** Protecting Groups. Georg Thieme Verlag, 1994
- **MORRISON et al.** *Adv. Enzymol. Relat. Areas Mol. Biol.,* 1988, vol. 61, 201
- **SIJWALI S. ; ROSENTHAL P. J.** *Proceedings of the National Academy of Sciences of the United States of America (PNAS,* 2004, vol. 101 (13), 4384-4389
- *Measurement of Parasite Growth rates and Inhibitor Sensitivity,* 4385
- **SINGH A. ; ROSENTHAL P. J.** *Antimicrobial Agents and Chemotherapy,* 2001, vol. 45 (3), 949-951
- **DIVO A. A. et al.** *Protozool,* 1985, vol. 32, 59-64